# EUROPEAN PATENT SPECIFICATION

(11) **EP 2 651 904 B1**
(45) Date of publication and mention of the grant of the patent: **12.11.2014**
(21) Application number: 11791571.0
(22) Date of filing: 07.12.2011
(51) Int. Cl.: C07D 239/26, C09K 11/06

(54) **BISPYRIMIDINES FOR ELECTRONIC APPLICATIONS**
BISPYRIMIDINE FÜR ELEKTRONISCHE ANWENDUNGEN
BISPYRIMIDINES COMME UTILISATIONS ELECTRONIQUES

(30) Priority: 13.12.2010 EP 10194757
(43) Date of publication of application: 23.10.2013
(73) Proprietor: BASF SE, 67056 Ludwigshafen (DE)
(72) Inventor: SCHÄFER, Thomas, 4410 Liestal (CH); WOLLEB, Heinz, 4232 Fehren (CH); SCHILDKNECHT, Christian, 68307 Mannheim (DE); WATANABE, Soichi, 68165 Mannheim (DE); LENNARTZ, Christian, 67105 Schifferstadt (DE)
(74) Representative: Leybach, Holger Hugo
(86) International application number: PCT/EP2011/072040
(87) International publication number: WO 2012/080052

(56) References cited:
- EP-A1- 1 724 323
- JP-A- 2003 045 662

## Description

The present invention relates to compounds of formula 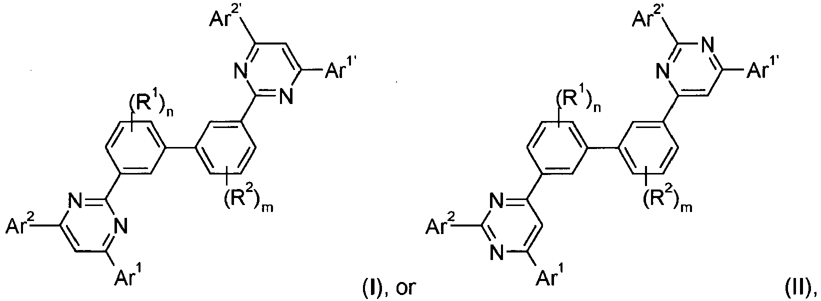 a process for their production and their use in electronic devices, especially electroluminescent devices. When used as electron transport material in electroluminescent devices, the compounds of formula I, or II may provide improved efficiency, stability, manufacturability, or spectral characteristics of electroluminescent devices.

EP-A-1,202,608 discloses EL devices comprising a carbazole compound of formula 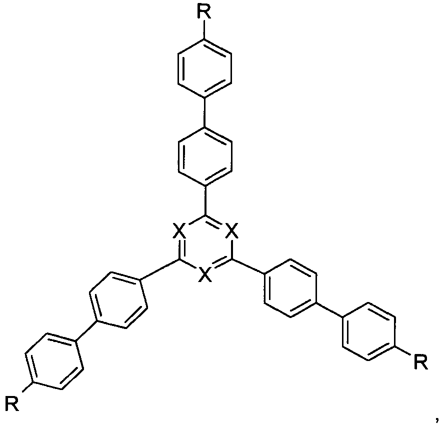 wherein R is 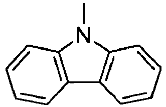 and X is C or
N, which constitutes the hole transporting layer.

JP2002324678 relates to light emitting elements comprising at least one kind of compound of formula, wherein

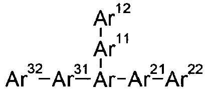

Ar¹¹, Ar²¹ and Ar³¹ denote arylene groups, Ar¹², Ar²² and Ar³² denote substituents or hydrogen atoms, wherein at least one of Ar¹¹, Ar²¹, Ar³⁴¹, Ar¹², Ar²² and Ar³² is either a condensed ring aryl structure or a condensed ring heteroaryl structure; Ar denotes an arylene group or a heteroarylene group; and at least one amine derivative having a condensed ring group with two or more rings are contained in a luminous layer. As examples of compounds of the above formula, wherein Ar denotes a heteroarylene group the following two compounds are explicitly mentioned: 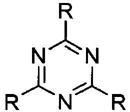 R is a group of formula 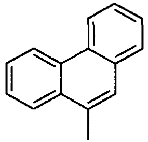 or 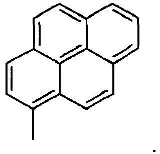 EP-A-926216 relates to EL devices using triaryl amine compounds, such as

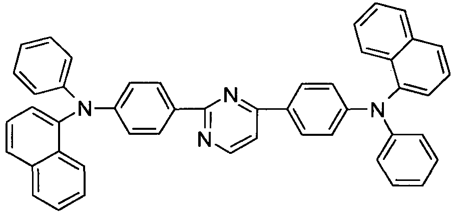

EP-A-690 053 relates to the use of conjugated compounds containing two or more pyrimidine rings, which are part of the conjugated system, as electroluminescent materials. The conjugated compounds described in EP-A-690 053 comprise pyrimidin-2,5-diyl groups which do not carry substituents at positions 4 and 6.

Hanan, Garry S. et al.,Canadian Journal of Chemistry (1997), 75(2), 169-182 disclose oligo-tridentate ligands based on alternating pyridines and pyrimidines, e.g. 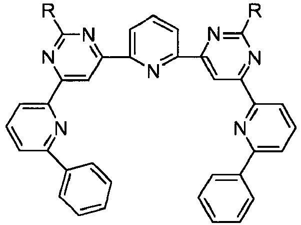 (R = H, Me, Ph), which were synthesized by Stille-type carbon-carbon bond-forming reactions. The terpyridine-like sites are designed to coalign upon metal complexation, giving rise to organized and rigidly spaced metal ions.

Shaker, Raafat M., Heteroatom Chemistry (2005), 16(6), 507-512 describes the synthesis of a series of 4,4'-(1,4-phenylene)-dipyrimidines, e.g., 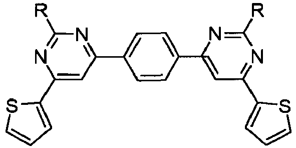 (R = Me, Ph or NH₂), by the reaction of amidines with the dienaminone, bis-chalcone, or ylidenemalononitrile.

JP2003045662 disclose compounds of the following formula 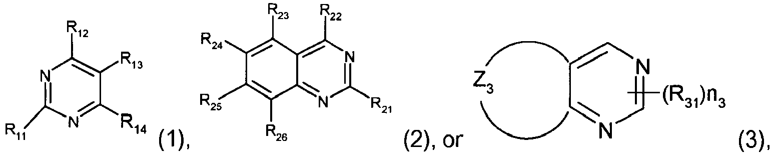 wherein R₁₁ to R₁₄ are H, or a monovalent substituent; at least one of R₁₁ to R₁₄ is an aromatic hydrocarbon group; R₂₁ to R₂₆ are H, or a monovalent substituent; R₃₁ is H, or a monovalent substituent; n3 = 0 - 2; Z₃ is a 5-membered ring.

The following "brigded" bispyrimidines are explicitly mentioned:

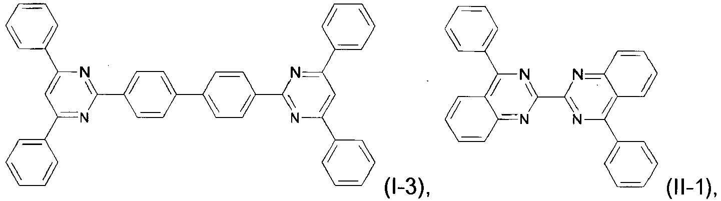

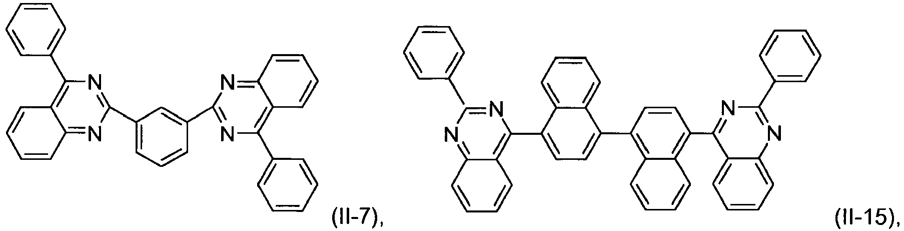

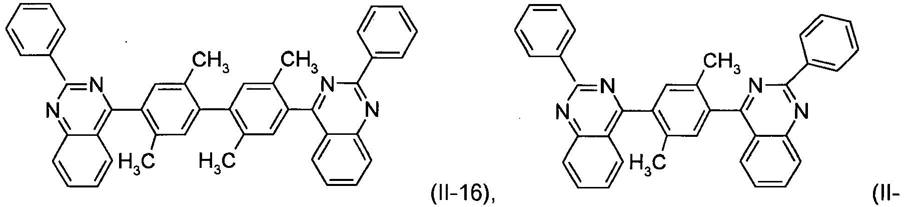

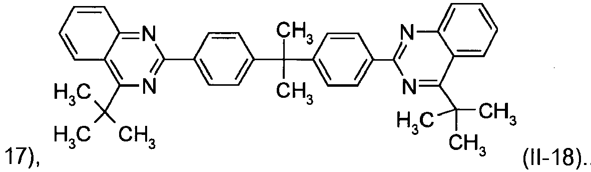

WO04039786 discloses bispyrimidines of formula 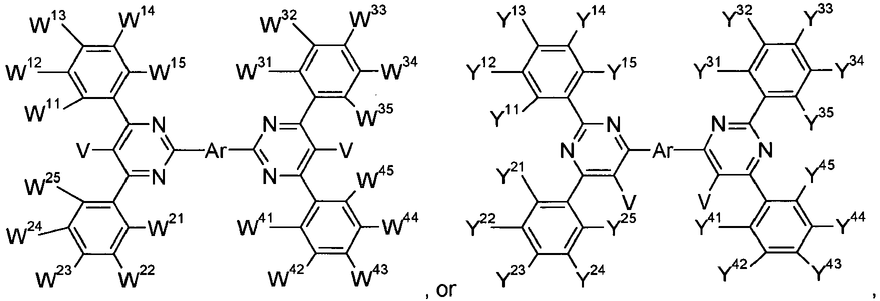 wherein
Ar is a group of formula 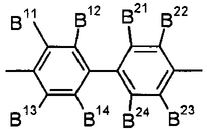 or 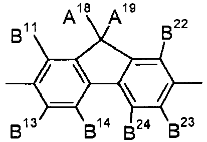 especially 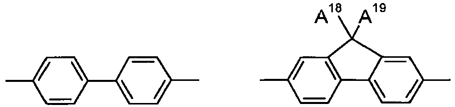 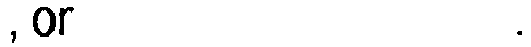 WO2008119666 discloses compounds of formula 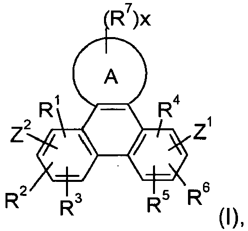 a process for their preparation and their use in organic light emitting diodes (OLEDs), especially as host for phosphorescent compounds. The hosts may function with phosphorescent materials to provide improved efficiency, stability, manufacturability, or spectral characteristics of electroluminescent devices. Z¹ and Z² can be a group of the formula 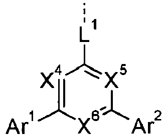 wherein X⁴, X⁵ and X⁶ are independently of each other N, or CH, with the proviso that at least one, preferably at least two of the substituents X⁴, X⁵ and X⁶ are N, and

Ar¹ and Ar² are independently of each other optionally substituted C₆-C₂₄aryl, or C₂-C₂₀heteroaryl. The following compound is explicitly disclosed:

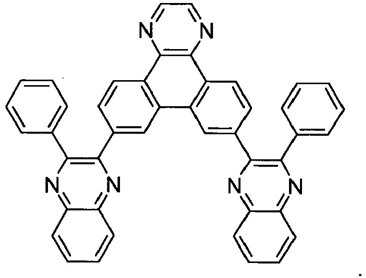

WO2009037155 discloses electroluminescent devices, comprising a compound of the formula 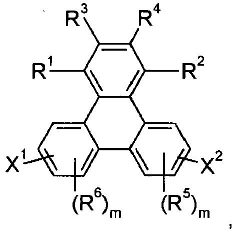 especially as host for phosphorescent compounds, wherein X¹ and X² can be a group of the formula 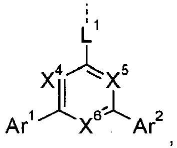 X⁴, X⁵ and X⁶ are independently of each other N, or CH, with the proviso that at least one, preferably at least two of the substituents X⁴, X⁵ and X⁶ are N, and Ar¹ and Ar² are independently of each other optionally substituted C₆-C₂₄aryl, or C₂-C₂₀heteroaryl. The hosts may function with phosphorescent materials to provide improved efficiency, stability, manufacturability, or spectral characteristics of electroluminescent devices.

WO2005053055 relates to EL devices, containing a hole blocking layer comprising a compound of the following structure 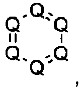 wherein Q is N or CR with the proviso that et least one Q is N, The following compounds are given as an example:

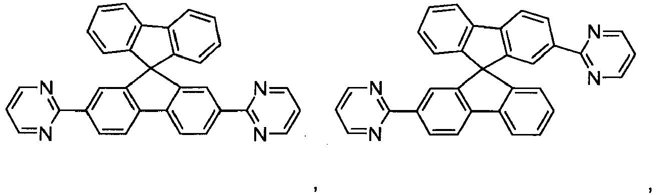

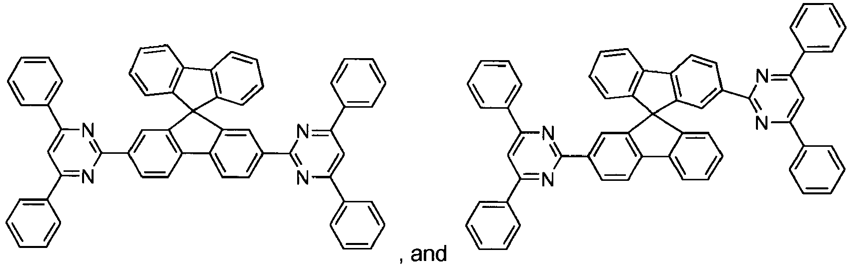

JP2009184987 describes compounds of the following structure for the use as electron transport materials: 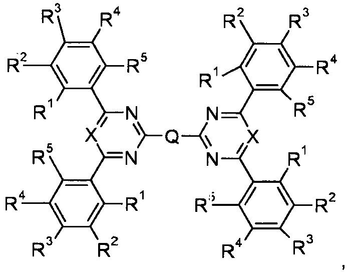 wherein X is CR, or N, and Q is

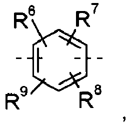

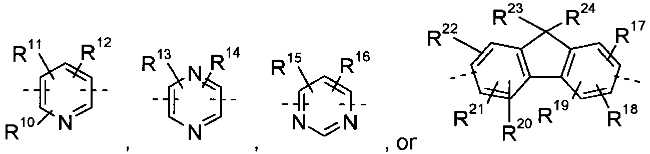

WO2009069442 relates to an organic electroluminescent device having high emission luminance and low driving voltage. The organic electroluminescent device contains at least one compound of formula 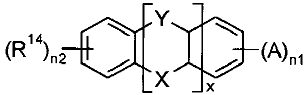 [A is a group of formula 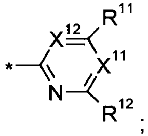 the sum of n1 and n2 is an integer of 6 to 8; X¹¹ and X¹² = N or CR¹³; R¹³ and R¹⁴ is H or substituent, but do not combine together to form a ring; when X¹¹ and X¹² are both CR¹³, both groups R¹³ may be the same as or different from each other; x is 0, or 1, when x = 1, -Y- and -X- are a direct bond or -O-, -S- or -N(R¹⁵)-; and R¹⁵ is a substituent].

Examples of such compounds are shown below:

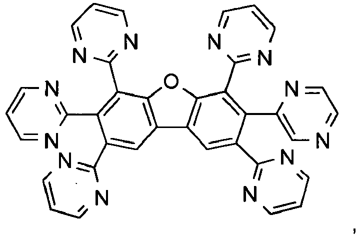

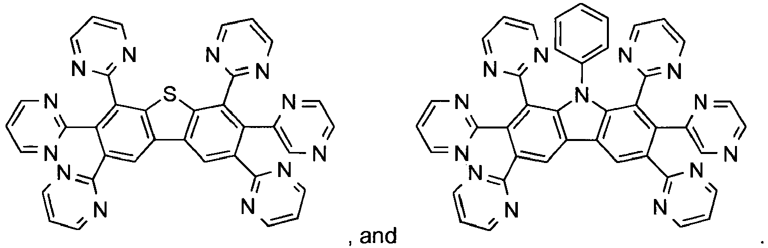

WO2009084546 describes an organic electroluminescent device (organic EL device), containing 2,2'-bispyrimidines of formula 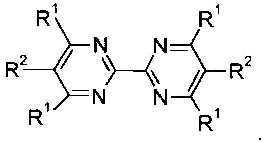 The light-emitting layer of the EL device contains a phosphorescent dopant and a bispyrimidine compound, such as, for example, 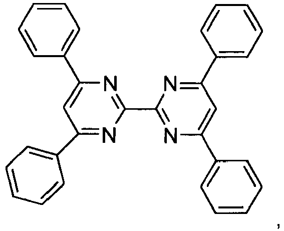 which serves as a host material.

WO2009054253 relates to an organic electroluminescent device. The organic electroluminescent device is characterized by containing at least one compound represented by the following general formula Ar-(A)ₙ (In the formulae, Ar represents a monocyclic or bicyclic aromatic ring or an aromatic heterocyclic ring, each of which contains at least one hydrogen atom; n represents an integer of 3, 4 or 5; A represents a group represented by the general formula 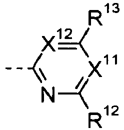 and a plurality of A's may be the same as or different from one another; X¹¹ and X¹² each represents a nitrogen atom or CR¹³; R¹¹, R¹² and R¹³ each represents a hydrogen atom or a substituent, but they do not combine together to form a ring; when X¹¹ and X¹² are both CR¹³, R¹³'s may be the same as or different from each other; and at least three of A's bonded to Ar are bonded to carbon atoms of Ar which are adjacent to one another.

US2009102356 discloses organic compounds having electron-transporting and/or hole-blocking performance, wherein said organic compound is a multi-aryl substituted pyridine derivative. An example of such a compound is shown below:

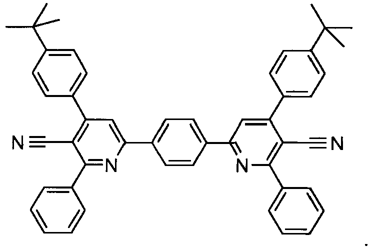

KR2009008737 describes compounds of the following structure as electron hole injection, electron injection and transport material for OLED. 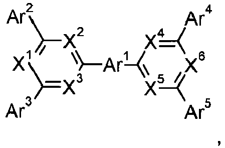 wherein X¹ to X⁶ are N, or CR. Examples of such compounds are shown below:

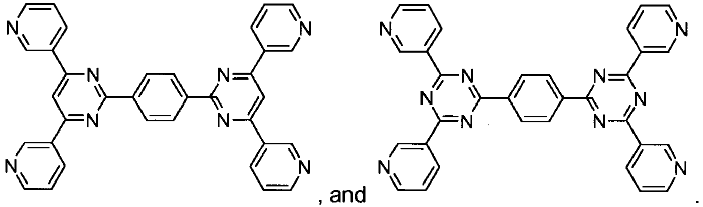

JP2009021336 relates to an organic electroluminescent element which has at least an anode, a light emission layer, an electron transporting layer, and a cathode as constitution layers such that the light emission layer contains at least a host compound and a metal complex. The host compound is a compound of formula 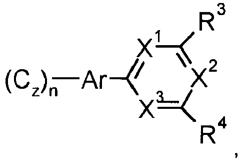 such as, for example,

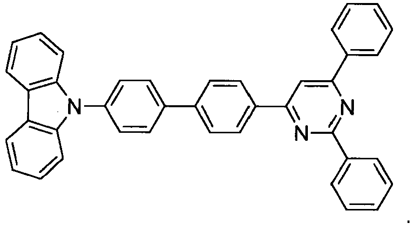

WO09008353 discloses an organic EL device containing a chrysene derivative, which has a larger energy gap than anthracenes, in an electron transport layer. The following bispyrimidine compounds are explicitly mentioned: 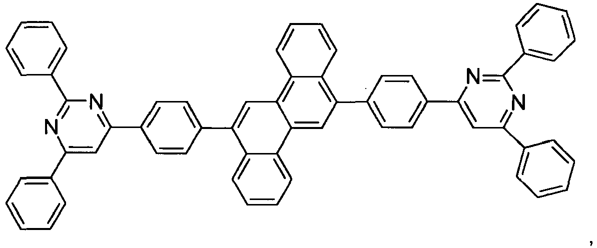 and

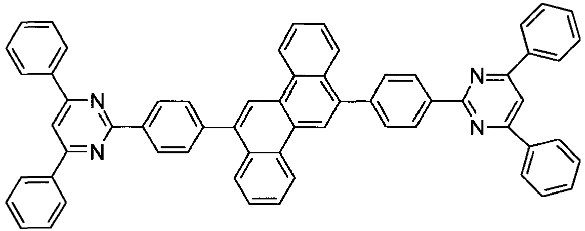

EP1724323 relates to an material for an organic electroluminescence device comprising a compound represented by the following general formula (1) : 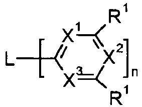 where: L represents a linking group having at least one meta bond; R¹ and R² each independently represent a hydrogen atom, an alkyl group which has 1 to 50 carbon atoms and which may have a substituent, a heterocyclic group which has 5 to 50 ring atoms and which may have a substituent, an alkoxy group which has 1 to 50 carbon atoms and which may have a substituent, an aryloxy group which has 5 to 50 ring carbon atoms and which may have a substituent, an aralkyl group which has 7 to 50 ring carbon atoms and which may have a substituent, an alkenyl group which has 2 to 50 carbon atoms and which may have a substituent, an alkylamino group which has 1 to 50 carbon atoms and which may have a substituent, an arylamino group which has 5 to 50 ring carbon atoms and which may have a substituent, an aralkylamino group which has 7 to 50 ring carbon atoms and which may have a substituent, an aryl group which has 6 to 50 ring carbon atoms and which may have a substituent, or a cyano group;

X¹ to X³ each independently represent =CR- or =N-, at least one of X¹ to X³ representing =N- where R represents an aryl group which has 6 to 50 ring carbon atoms and which may have a substituent, a heterocyclic group which has 5 to 50 ring atoms and which may have a substituent, an alkyl group which has 1 to 50 carbon atoms and which may have a substituent, an alkoxy group which has 1 to 50 carbon atoms and which may have a substituent, an aralkyl group which has 7 to 50 ring carbon atoms and which may have a substituent, an aryloxy group which has 5 to 50 ring carbon atoms and which may have a substituent, an arylthio group which has 5 to 50 ring carbon atoms and which may have a substituent, a carboxyl group, a halogen atom, a cyano group, a nitro group, or a hydroxyl group; and
n represents an integer of 1 to 5. The following bispyrimidine compounds are explicitly disclosed: 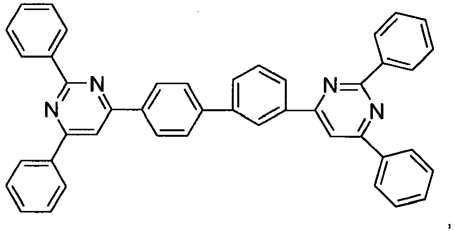 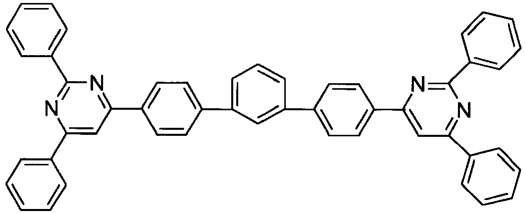 etc. The compound of formula (1) is preferably used as host material in combination with a phosphorescent dopant in the light emitting layer.

JP2005255561 provides a selective method for producing a multi-substituted pyrimidine suitable for a luminescent material, such as, for example,

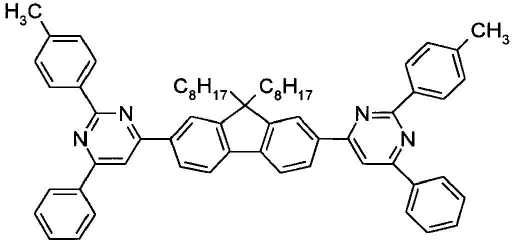

WO10090077 relates to an material for an organic electroluminescence device comprising a compound represented by the following general formula 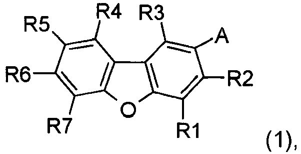 such as, for example,

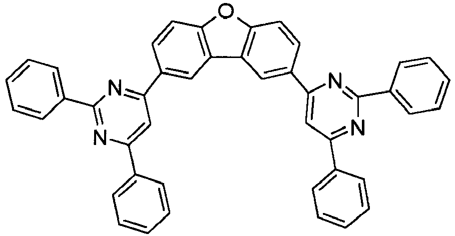

The following compound is explicitly disclosed in JP2010135467:

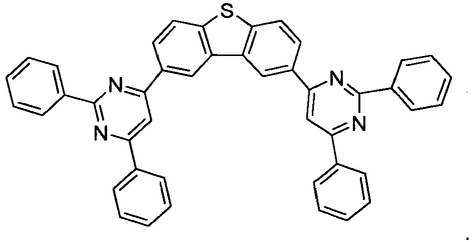

Notwithstanding these developments, there remains a need for organic light emitting devices comprising new electron transport materials to provide improved efficiency, stability, manufacturability, and/or spectral characteristics of electroluminescent devices.

Accordingly, it was the object of the present invention to provide compounds, which when used in organic electronic devices, especially organic light emitting devices showing good efficiencies, good operative lifetimes, good manufacturability, good spectral characteristics, a high stability to thermal stress, and/or a low operating voltage.

Certain organic compounds containing two pyrimidine moieties are found to be suitable for use in organo-electroluminescent devices. In particular, certain pyrimidine derivatives are suitable electron transporting materials, or hole blocking materials with good efficiency and durability. When the compounds are used as electron transporting material, they may also prevent holes or excitons from entering the electron transport layer.

Said object has been solved by compounds of the formula 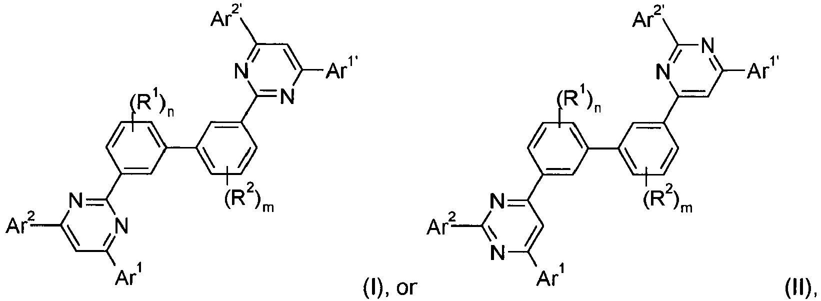 wherein
Ar¹, Ar², Ar^{1'} and Ar^{2'} are independently of each other a C₆-C₂₄aryl group, or a C₂-C₃₀heteroaryl group, which can optionally be substituted by G, a C₄-C₁₈cycloalkyl, a C₆-C₁₀aryl group, a C₆-C₁₀aryl group which is substituted by by C₁-C₈alkyl, a C₂-C₅heteroaryl group, or a C₂-C₅heteroaryl group, which is substituted by by C₁-C₈alkyl,
R¹ and R² can be the same or different at each occurrence and are F, CN, NR⁴⁵R^{45'}, a C₁-C₂₅alkyl group, a C₄-C₁₈cycloalkyl group, a C₁-C₂₅alkoxy group which is substituted by E or interrupted by D, a C₆-C₂₄aryl group, a C₆-C₂₄aryl group which is substituted by G, a C₂-C₃₀heteroaryl group, or a C₂-C₃₀heteroaryl group, which is substituted by G,
m and n are 0, 1, 2, 3, or 4,
D is -CO-, -COO-, -S-, -SO-, -SO₂-, -O-,-NR⁶⁵-, -SiR⁷⁰R⁷¹-, -POR⁷²-, -CR⁶³=CR⁶⁴-, or -C≡C-, E is -OR⁶⁹, -SR⁶⁹, -NR⁶⁵R⁶⁶, -COR⁶⁸, -COOR⁶⁷, -CONR⁶⁵R⁶⁶, -CN, or halogen,
G is E, or C₁-C₂₅alkyl,
R⁴⁵ and R⁴s' are independently of each other a C₁-C₂₅alkyl group, a C₄-C₁₈cycloalkyl group, in which one or more carbon atoms which are not in neighbourhood to each other could be replaced by -NR^{45"}-, -O-, -S-, -C(=O)-O-, or, -O-C(=O)-O-, and/or wherein one or more hydrogen atoms can be replaced by F, a C₆-C₂₄aryl group, or a C₆-C₂₄aryloxy group, wherein one or more carbon atoms can be replaced by O, S, or N, and/or which can be substituted by one or more non-aromatic groups R¹, and
R^{45"} is a C₁-C₂₅alkyl group, or a C₄-C₁₈cycloalkyl group,
R⁶³ and R⁶⁴ are independently of each other H, C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-;
R⁶⁵ and R⁶⁶ are independently of each other C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-; or R⁶⁵ and R⁶⁶ together form a five or six membered ring, or ring system;
R⁶⁷ is C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-,
R⁶⁸ is H; C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-,
R⁶⁹ is C₆-C₁₈aryl; C₆-C₁₈aryl, which is substituted by C₁-C₁₈alkyl, or C₁-C₁₈alkoxy; C₁-C₁₈alkyl; or C₁-C₁₈alkyl which is interrupted by -O-,
R⁷⁰ and R⁷¹ are independently of each other C₁-C₁₈alkyl, C₆-C₁₈aryl, or C₆-C₁₈aryl, which is substituted by C₁-C₁₈alkyl, and
R⁷² is C₁-C₁₈alkyl, C₆-C₁₈aryl, or C₆-C₁₈aryl, which is substituted by C₁-C₁₈alkyl.
R¹ and R² are preferably methyl, ethyl, propyl, iso-propyl, n-butyl, t-putyl, or phenyl.
m and n are preferably 0.

In a preferred embodiment the present invention is directed to compounds of formula 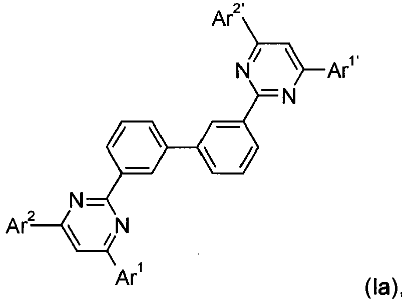 wherein Ar¹, Ar², Ar^{1'} and Ar^{2'} are as defined above.

Preferably, Ar¹, Ar², Ar^{1'} and Ar^{2'} are independently of each other selected from 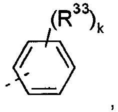 and 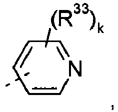 wherein

R³³ is a C₁-C₂₅alkyl group, a C₄-C₁₈cycloalkyl group, a C₁-C₂₅alkoxy group, a C₆-C₁₀aryl group, a C₆-C₁₀aryl group which is substituted by by C₁-C₈alkyl, a C₂-C₅heteroaryl group, or a C₂-C₅heteroaryl group, which is substituted by by C₁-C₈alkyl, and k is 0, 1,2, 3, or 4.

Preferably R³³ is C₁-C₈alkyl group, a C₆-C₁₀aryl group, a C₆-C₁₀aryl group which is substituted by C₁-C₈alkyl, a C₂-C₅heteroaryl group, or a C₂-C₅heteroaryl group, which is substituted by C₁-C₈alkyl.

k is preferably 0, 1, or 2, most preferred 0.

More preferably, Ar¹, Ar², Ar^{1'} and Ar^{2'} are independently of each other selected from

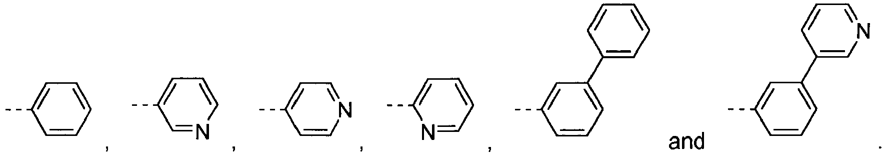

Compounds **B-1** to **B-8** are particularly preferred. Reference is made to claim 7.

In another preferred embodiment the present invention is directed to compounds of formula 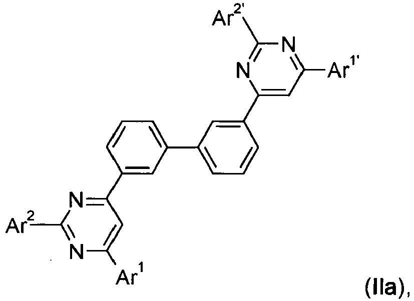 wherein Ar¹, Ar², Ar^{1'} and Ar^{2'} are as defined in claim 1.

Preferably, Ar¹, Ar², Ar^{1'} and Ar^{2'} are independently of each other selected from 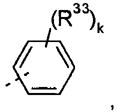 and 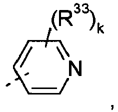 wherein R³³ is a C₁-C₂₅alkyl group, a C₄-C₁₈cycloalkyl group, a C₆-C₁₀aryl group, a C₆-C₁₀aryl group which is substituted by by C₁-C₈alkyl, a C₂-C₅heteroaryl group, or a C₂-C₅heteroaryl group, which is substituted by by C₁-C₈alkyl, and k is 0, 1, 2, 3, or 4.

More preferably, Ar¹, Ar², Ar^{1'} and Ar^{2'} are independently of each other selected from

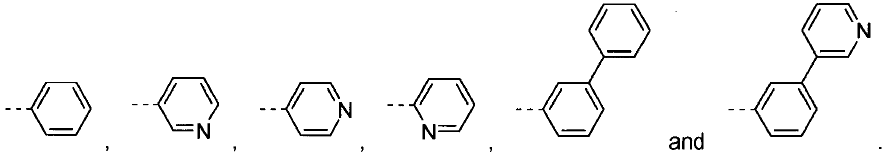

Compounds **A-1** to **A-8** are particularly preferred. Reference is made to claim 4.

The compounds of formula I of the present invention can be prepared according to a process, which comprises reacting a compound of formula 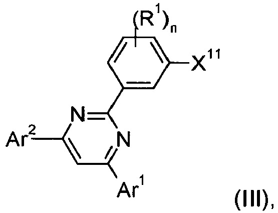 wherein X¹¹ stands for halogen, such as bromo, or iodo, with a compound of formula 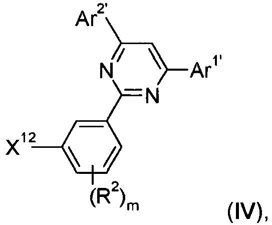 wherein X¹² is -B(OH)₂, -B(OY¹)₂, 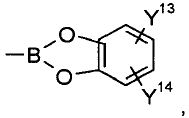 or 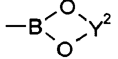 wherein Y¹ is independently in each occurrence a C₁-C₁₈alkylgroup and Y² is independently in each occurrence a C₂-C₁₀alkylene group, such as -CY³Y⁴-CY⁵Y⁶-, or - CY⁷Y⁸-CY⁹Y¹⁰- CY¹¹Y¹²-, wherein Y3, Y⁴, Y⁵, Y⁶, Y⁷, Y⁸, Y⁹, Y¹⁰, Y¹¹ and Y¹² are independently of each other hydrogen, or a C₁-C₁₈alkylgroup, especially -C(CH₃)₂C(CH₃)₂-, -C(CH₃)₂CH₂C(CH₃)₂-, or -CH₂C(CH₃)₂CH₂-, and Y¹³ and Y¹⁴ are independently of each other hydrogen, or a C₁-C₁₈alkylgroup, in the presence of a base and a catalyst in a solvent, wherein Ar¹, Ar¹, Ar², Ar^{2'}, m, n, R¹ and R² are as defined above.

The compounds of formula II of the present invention can be prepared according to a process, which comprises reacting a compound of formula 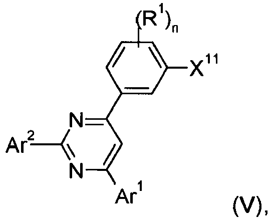 wherein X¹¹ stands for halogen, such as bromo, or iodo, with a compound of formula 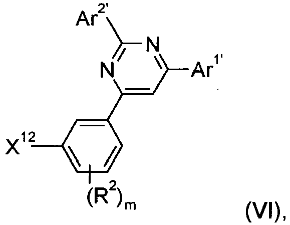 wherein X¹² is -B(OH)₂, -B(OY¹)₂, 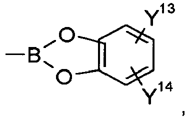 or 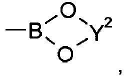 wherein Y¹ is independently in each occurrence a C₁-C₁₈alkylgroup and Y² is independently in each occurrence a C₂-C₁₀alkylene group, such as -CY³Y⁴-CY⁵Y⁶-, or - CY⁷Y⁸-CY⁹Y¹⁰- CY¹¹Y¹²-, wherein Y³, Y⁴, Y⁵, Y⁶, Y⁷, Y⁸, Y⁹, Y¹⁰, Y¹¹ and Y¹² are independently of each other hydrogen, or a C₁-C₁₈alkylgroup, especially -C(CH₃)₂C(CH₃)₂-, -C(CH₃)₂CH₂C(CH₃)₂-, or -CH₂C(CH₃)₂CH₂-, and Y¹³ and Y¹⁴ are independently of each other hydrogen, or a C₁-C₁₈alkylgroup, in the presence of a base and a catalyst in a solvent, wherein Ar¹, Ar^{1'}, Ar², Ar^{2'}, m, n, R¹ and R² are as defined above. The catalyst may be one of the µ-halo(triisopropylphosphine)(η³-allyl)palladium(II) type (see for example WO99/47474).

Preferably, the reaction is carried out in the presence of an organic solvent, such as an aromatic hydrocarbon or a usual polar organic solvent, such as benzene, toluene, xylene, tetrahydrofurane, or dioxane, or mixtures thereof, most preferred toluene. Usually, the amount of the solvent is chosen in the range of from 1 to 10 I per mol of boronic acid derivative. Also preferred, the reaction is carried out under an inert atmosphere such as nitrogen, or argon. Further, it is preferred to carry out the reaction in the presence of an aqueous base, such as an alkali metal hydroxide or carbonate such as NaOH, KOH, Na₂CO₃, K₂CO₃, Cs₂CO₃ and the like, preferably an aqueous K₂CO₃ solution is chosen. Usually, the molar ratio of the base to boronic acid or boronic ester derivative is chosen in the range of from 0.5:1 to 50:1, very especially 1:1. Generally, the reaction temperature is chosen in the range of from 40 to 180°C, preferably under reflux conditions. Preferred, the reaction time is chosen in the range of from 1 to 80 hours, more preferably from 20 to 72 hours. In a preferred embodiment a usual catalyst for coupling reactions or for polycondensation reactions is used, preferably Pd-based, which is described in WO2007/101820. The palladium compound is added in a ratio of from 1:10000 to 1:50, preferably from 1:5000 to 1:200, based on the number of bonds to be closed. Preference is given, for example, to the use of palladium(II) salts such as PdAc₂ or Pd₂dba₃ and to the addition of ligands selected from the
group consisting of 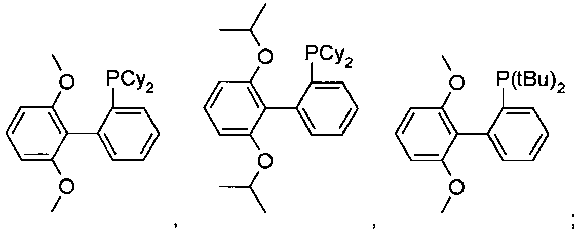 wherein

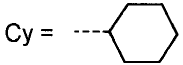

The ligand is added in a ratio of from 1:1 to 1:10, based on Pd. Also preferred, the catalyst is added in solution, or suspension. Preferably, an appropriate organic solvent, such as the ones described above, preferably benzene, toluene, xylene, THF, dioxane, more preferably toluene, or mixtures thereof, is used. The amount of solvent usually is chosen in the range of from 1 to 10 I per mol of boronic acid derivative.

The synthesis of pyrimidines carrying bromine units, i.e. the compounds of formula III and **V,** can be done in analogy to the methods described in US20070190355 and Majid M. Heravi et al., Tetrahedron Letters 50 (2009) 662-666.

Compounds of formula **IV** and **VI** can be obtained by reacting compounds of formula III and **V** with (OY¹)₂B-B(OY¹)₂, 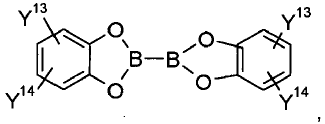 or 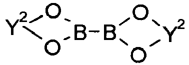 in the presence of a catalyst, such as, for example, [1,1'-bis(diphenylphosphino)ferrocene]dichloro-palladium(II), complex (Pd(Cl)₂(dppf)), and a base, such as, for example, potassium acetate, in a solvent, such as, for example, dimethyl formamide, dimethyl sulfoxide, dioxane and/or toluene (cf. Prasad Appukkuttan et al., Synlett 8 (2003) 1204).

The compounds of of the present invention may be used for electrophotographic photoreceptors, photoelectric converters, organic solar cells (organic photovoltaics), switching elements, such as organic transistors, for example, organic FETs and organic TFTs, organic light emitting field effect transistors (OLEFETs), image sensors, dye lasers and electroluminescent devices (=organic light-emitting diodes (OLEDs)).

Accordingly, a further subject of the present invention is directed to an electronic device, comprising a compound according to the present invention.

The electronic device is preferably an electroluminescent device.

The compounds of formula I, or II, can in principal be used in any layer of an EL device, but are preferably used as electron transport material.

Hence, a further subject of the present invention is directed to an electron transport layer, comprising a compound of the present invention.

Halogen is fluorine, chlorine, bromine and iodine.

C₁-C₂₅alkyl (C₁-C₁₈alkyl) is typically linear or branched, where possible. Examples are methyl, ethyl, n-propyl, isopropyl, n-butyl, sec.-butyl, isobutyl, tert.-butyl, n-pentyl, 2-pentyl, 3-pentyl, 2,2-dimethylpropyl, 1,1,3,3-tetramethylpentyl, n-hexyl, 1-methylhexyl, 1,1,3,3,5,5-hexamethylhexyl, n-heptyl, isoheptyl, 1,1,3,3-tetramethylbutyl, 1-methylheptyl, 3-methylheptyl, n-octyl, 1,1,3,3-tetramethylbutyl and 2-ethylhexyl, n-nonyl, decyl, undecyl, dodecyl, tridecyl, tetradecyl, pentadecyl, hexadecyl, heptadecyl, or octadecyl. C₁-C₈alkyl is typically methyl, ethyl, n-propyl, isopropyl, n-butyl, sec.-butyl, isobutyl, tert.-butyl, n-pentyl, 2-pentyl, 3-pentyl, 2,2-dimethyl-propyl, n-hexyl, n-heptyl, n-octyl, 1,1,3,3-tetramethylbutyl and 2-ethylhexyl. C₁-C₄alkyl is typically methyl, ethyl, n-propyl, isopropyl, n-butyl, sec.-butyl, isobutyl, tert.-butyl.

C₁-C₂₅alkoxy groups (C₁-C₁₈alkoxy groups) are straight-chain or branched alkoxy groups, e.g. methoxy, ethoxy, n-propoxy, isopropoxy, n-butoxy, sec-butoxy, tert-butoxy, amyloxy, isoamyloxy or tert-amyloxy, heptyloxy, octyloxy, isooctyloxy, nonyloxy, decyloxy, undecyloxy, dodecyloxy, tetradecyloxy, pentadecyloxy, hexadecyloxy, heptadecyloxy and octadecyloxy. Examples of C₁-C₈alkoxy are methoxy, ethoxy, n-propoxy, isopropoxy, n-butoxy, sec.-butoxy, isobutoxy, tert.-butoxy, n-pentyloxy, 2-pentyloxy, 3-pentyloxy, 2,2-dimethylpropoxy, n-hexyloxy, n-heptyloxy, n-octyloxy, 1,1,3,3-tetramethylbutoxy and 2-ethylhexyloxy, preferably C₁-C₄alkoxy such as typically methoxy, ethoxy, n-propoxy, isopropoxy, n-butoxy, sec.-butoxy, isobutoxy, tert.-butoxy.

The term "cycloalkyl group" is typically C₄-C₁₈cycloalkyl, especially C₅-C₁₂cycloalkyl, such as cyclopentyl, cyclohexyl, cycloheptyl, cyclooctyl, cyclononyl, cyclodecyl, cycloundecyl, cyclododecyl, preferably cyclopentyl, cyclohexyl, cycloheptyl, or cyclooctyl, which may be unsubstituted or substituted.

C₆-C₂₄aryl (C₆-C₁₈aryl), which optionally can be substituted, is typically phenyl, 4-methylphenyl, 4-methoxyphenyl, naphthyl, especially 1-naphthyl, or 2-naphthyl, biphenylyl, terphenylyl, pyrenyl, 2- or 9-fluorenyl, phenanthryl, or anthryl, which may be unsubstituted or substituted.

C₂-C₃₀heteroaryl represents a ring with five to seven ring atoms or a condensed ring system, wherein nitrogen, oxygen or sulfur are the possible hetero atoms, and is typically a heterocyclic group with five to 30 atoms having at least six conjugated π-electrons such as thienyl, benzothiophenyl, dibenzothiophenyl, thianthrenyl, furyl, furfuryl, 2H-pyranyl, benzofuranyl, isobenzofuranyl, dibenzofuranyl, phenoxythienyl, pyrrolyl, imidazolyl, pyrazolyl, pyridyl, bipyridyl, triazinyl, pyrimidinyl, pyrazinyl, pyridazinyl, indolizinyl, isoindolyl, indolyl, indazolyl, purinyl, quinolizinyl, chinolyl, isochinolyl, phthalazinyl, naphthyridinyl, chinoxalinyl, chinazolinyl, cinnolinyl, pteridinyl, carbazolyl, carbolinyl, benzotriazolyl, benzoxazolyl, phenanthridinyl, acridinyl, pyrimidinyl, phenanthrolinyl, phenazinyl, isothiazolyl, phenothiazinyl, isoxazolyl, furazanyl or phenoxazinyl, which can be unsubstituted or substituted.

The C₆-C₂₄aryl (C₆-C₁₈aryl) and C₂-C₃₀heteroaryl groups are preferably substituted by one, or more C₁-C₈alkyl groups.

The term "aryl ether group" is typically a C₆-₂₄aryloxy group, that is to say O-C₆₋₂₄aryl, such as, for example, phenoxy or 4-methoxyphenyl.

Possible substituents of the above-mentioned groups are C₁-C₈alkyl, a hydroxyl group, a mercapto group, C₁-C₈alkoxy, C₁-C₈alkylthio, halogen, halo-C₁-C₈alkyl, or a cyano group.

If a substituent, such as, for example R¹ occurs more than one time in a group, it can be different in each occurrence.

The wording "substituted by G" means that one, or more, especially one to three substituents G might be present.

As described above, the aforementioned groups may be substituted by E and/or, if desired, interrupted by D. Interruptions are of course possible only in the case of groups containing at least 2 carbon atoms connected to one another by single bonds; C₆-C₁₈aryl is not interrupted; interrupted arylalkyl contains the unit D in the alkyl moiety. C₁-C₁₈alkyl substituted by one or more E and/or interrupted by one or more units D is, for example, (CH₂CH₂O)₁₋₉-R^{x}, where R^{x} is H or C₁-C₁₀alkyl or C₂-C₁₀alkanoyl (e.g. CO-CH(C₂H₅)C₄H₉), CH₂-CH(OR^{y'})-CH₂-O-R^{y}, where R^{y} is C₁-C₁₈alkyl, C₅-C₁₂cycloalkyl, phenyl, C₇-C₁₅phenylalkyl, and R^{y'} embraces the same definitions as R^{y} or is H;

C₁-C₈alkylene-COO-R^{z}, e.g. CH₂COOR^{z}, CH(CH₃)COOR^{z}, C(CH₃)₂COOR^{z}, where R^{z} is H, C₁-C₁₈alkyl, (CH₂CH₂O)₁₋₉-R^{x}, and R^{x} embraces the definitions indicated above; CH₂CH₂-O-CO-CH=CH₂; CH₂CH(OH)CH₂-O-CO-C(CH₃)=CH₂.

The organic electronic device of the present application is, for example, an organic solar cell (organic photovoltaics), a switching element. such as an organic transistors, for example organic FET and organic TFT, organic light emitting field effect transistor (OLEFET), or an organic light-emitting diode (OLED), preference being given to OLEDs.

The present application relates to the use of the compounds of formula I, or II preferably as electron transport layer in an organic electronic device.

Accordingly, the present application is also directed to an organic layer, especially an electron transport layer, comprising a compound of formula I, or II.

Suitable structures of organic electronic devices are known to those skilled in the art and are specified below.

The organic transistor generally includes a semiconductor layer formed from an organic layer with hole transport capacity and/or electron transport capacity; a gate electrode formed from a conductive layer; and an insulating layer introduced between the semiconductor layer and the conductive layer. A source electrode and a drain electrode are mounted on this arrangement in order thus to produce the transistor element. In addition, further layers known to those skilled in the art may be present in the organic transistor.

The organic solar cell (photoelectric conversion element) generally comprises an organic layer present between two plate-type electrodes arranged in parallel. The organic layer may be configured on a comb-type electrode. There is no particular restriction regarding the site of the organic layer and there is no particular restriction regarding the material of the electrodes. When, however, plate-type electrodes arranged in parallel are used, at least one electrode is preferably formed from a transparent electrode, for example an ITO electrode or a fluorine-doped tin oxide electrode. The organic layer is formed from two sublayers, i.e. a layer with p-type semiconductor properties or hole transport capacity, and a layer formed with n-type semiconductor properties or electron transport capacity. In addition, it is possible for further layers known to those skilled in the art to be present in the organic solar cell. The layer with electron transport capacity may comprise the compounds of formula I, or II.

The present invention further relates to an organic light-emitting diode comprising an anode An and a cathode Ka, a light-emitting layer E arranged between the anode An and the cathode Ka, an electron transport layer arranged between the cathode Ka and the light-emitting layer E, and if appropriate at least one further layer selected from the group consisting of at least one blocking layer for holes/excitons, at least one blocking layer for electrons/excitons, at least one hole injection layer, at least one hole transport layer and at least one electron injection layer, wherein the electron transport layer comprises a compound of formula I, or II.

### Structure of the inventive OLED

The inventive organic light-emitting diode (OLED) thus generally has the following structure:
an anode (An) and a cathode (Ka) and a light-emitting layer E arranged between the anode (An) and the cathode (Ka) and an electron transport layer arranged between the cathode Ka and the light-emitting layer E.

The inventive OLED may, for example - in a preferred embodiment - be formed from the following layers:
1. Anode
2. Hole transport layer
3. Light-emitting layer
4. Blocking layer for holes/excitons
5. Electron transport layer
6. Cathode

Layer sequences different than the aforementioned structure are also possible, and are known to those skilled in the art. For example, it is possible that the OLED does not have all of the layers mentioned; for example, OLEDs which have layers (1), (3), (4), (5) and (6), are likewise suitable. In addition, the OLEDs may have a blocking layer for electrons/excitons between the hole transport layer (2) and the light-emitting layer (3).

It is additionally possible that a plurality of the aforementioned functions (electron/exciton blocker, hole/exciton blocker, hole injection, hole transport, electron injection, electron transport) are combined in one layer and are assumed, for example, by a single material present in this layer. For example, a material used in the hole transport layer, in one embodiment, may simultaneously block excitons and/or electrons.

Furthermore, the individual layers of the OLED among those specified above may in turn be formed from two or more layers. For example, the hole transport layer may be formed from a layer into which holes are injected from the electrode, and a layer which transports the holes away from the hole-injecting layer into the light-emitting layer. The electron transport layer may likewise consist of a plurality of layers, for example a layer in which electrons are injected by the electrode, and a layer which receives electrons from the electron injection layer and transports them into the light-emitting layer. These layers mentioned are each selected according to factors such as energy level, thermal resistance and charge carrier mobility, and also energy difference of the layers specified with the organic layers or the metal electrodes. The person skilled in the art is capable of selecting the structure of the OLEDs such that it is matched optimally to the organic compounds used as emitter substances in accordance with the invention.

In order to obtain particularly efficient OLEDs, for example, the HOMO (highest occupied molecular orbital) of the hole transport layer should be matched to the work function of the anode, and the LUMO (lowest unoccupied molecular orbital) of the electron transport layer should be matched to the work function of the cathode, provided that the aforementioned layers are present in the inventive OLEDs.

The anode (1) is an electrode which provides positive charge carriers. It may be formed, for example, from materials which comprise a metal, a mixture of various metals, a metal alloy, a metal oxide or a mixture of various metal oxides. Alternatively, the anode may be a conductive polymer. Suitable metals comprise metals and alloys of the metals of the main groups, transition metals and of the lanthanoids, especially the metals of groups Ib, IVa, Va and VIa of the periodic table of the elements, and the transition metals of group VIIIa. When the anode is to be transparent, generally mixed metal oxides of groups IIb, IIIb and IVb of the periodic table of the elements (IUPAC version) are used, for example indium tin oxide (ITO). It is likewise possible that the anode (1) comprises an organic material, for example polyaniline, as described, for example, in Nature, Vol. 357, pages 477 to 479 (June 11, 1992). At least either the anode or the cathode should be at least partly transparent in order to be able to emit the light formed. The material used for the anode (1) is preferably ITO.

Suitable hole transport materials for layer (2) of the inventive OLEDs are disclosed, for example, in Kirk-Othmer Encyclopedia of Chemical Technology, 4th edition, Vol. 18, pages 837 to 860, 1996. Both hole-transport molecules and polymers can be used as the hole transport material. Hole-transport molecules typically used are selected from the group consisting of tris[N-(1-naphthyl)-N-(phenylamino)]triphenylamine (1-NaphDATA), 4,4'-bis[N-(1-naphthyl)-N-phenylamino]biphenyl (α-NPD), N,N'-diphenyl-N,N'-bis(3-methylphenyl)-[1,1'-biphenyl]-4,4'-diamine (TPD), 1,1-bis[(di-4-tolylamino)phenyl]cyclohexane (TAPC), N,N'-bis(4-methylphenyl)-N,N'-bis(4-ethylphenyl)-[1,1'-(3,3'-dimethyl)biphenyl]-4,4'-diamine (ETPD), tetrakis(3-methylphenyl)-N,N,N',N'-2,5-phenylenediamine (PDA), α-phenyl-4-N,N-diphenylaminostyrene (TPS), p-(diethylamino)benzaldehyde diphenylhydrazone (DEH), triphenylamine (TPA), bis[4-(N,N-diethylamino)-2-methylphenyl)(4-methylphenyl)methane (MPMP), 1-phenyl-3-[p-(diethylamino)styryl]-5-[p-(diethylamino)phenyl]pyrazoline (PPR or DEASP), 1 ,2-trans-bis(9H-carbazol-9-yl)cyclobutane (DCZB), N,N,N',N'-tetrakis(4-methylphenyl)-(1,1'-biphenyl)-4,4'-diamine (TTB), 4,4',4"-tris(N,N-diphenylamino)triphenylamine (TDTA), 4,4',4"-tris(N-carbazolyl)triphenylamine (TCTA), N,N'-bis(naphthalen-2-yl)-N,N'-bis(phenyl)benzidine (β-NPB), N,N'-bis(3-methylphenyl)-N,N'-bis(phenyl)-9,9-spirobifluorene (Spiro-TPD), N,N'-bis(naphthalen-1-yl)-N,N'-bis(phenyl)-9,9-spirobifluorene (Spiro-NPB), N,N'-bis(3-methylphenyl)-N,N'-bis(phenyl)-9,9-dimethylfluorene (DMFL-TPD), di[4-(N,N-ditolylamino)phenyl]cyclohexane, N,N'-bis(naphthalen-1-yl)-N,N'-bis(phenyl)-9,9-dimethylfluorene, N,N'-bis(naphthalen-1-yl)-N,N'-bis(phenyl)-2,2-dimethylbenzidine, N,N'-bis(naphthalen-1-yl)-N,N'-bis(phenyl)benzidine, N,N'-bis(3-methylphenyl)-N,N'-bis(phenyl)benzidine, 2 , 3 , 5 , 6-tetrafluoro-7,7,8,8-tetracyanoquinodimethane (F4-TCNQ), 4,4',4"-tris(N-3-methylphenyl-N-phenylamino)triphenylamine, 4,4',4"-tris(N-(2-naphthyl)-N-phenyl-amino)triphenylamine, pyrazino[2,3-f][1,10]phenanthroline-2,3-dicarbonitrile (PPDN), N,N,N',N'-tetrakis(4-methoxyphenyl)benzidine (MeO-TPD), 2,7-bis[N,N-bis(4-methoxyphenyl)amino]-9,9-spirobifluorene (MeO-Spiro-TPD), 2,2'-bis[N,N-bis(4-methoxyphenyl)amino]-9,9-spirobifluorene (2,2'-MeO-Spiro-TPD), N,N'-diphenyl-N,N'-di[4-(N,N-ditolylamino)phenyl]benzidine (NTN PB), N, N'-diphenyl-N,N'-di[4-(N, N-diphenylamino)phenyl]benzidine (NPNPB), N,N'-di(naphthalen-2-yl)-N,N'-diphenylbenzene-1,4-diamine (β-NPP), N,N'-bis(3-methylphenyl)-N,N'-bis(phenyl)-9,9-diphenylfluorene (DPFL-TPD), N,N'-bis(naphthalen-1-yl)-N,N'-bis(phenyl)-9,9-diphenylfluorene (DPFL-NPB), 2,2',7,7'-tetrakis(N,N-diphenylamino)-9,9'-spirobifluorene (Spiro-TAD), 9,9-bis[4-(N,N-bis(biphenyl-4-yl)amino)phenyl]-9H-fluorene (BPAPF), 9,9-bis[4-(N,N-bis(naphthalen-2-yl)amino)phenyl]-9H-fluorene (NPAPF), 9,9-bis[4-(N,N-bis(naphthalen-2-yl)-N,N'-bisphenylamino)phenyl]-9H-fluorene (NPBAPF), 2,2',7,7 '-tetrakis[N-naphthalenyl(phenyl)amino]-9,9'-spirobifluorene (Spiro-2NPB), N,N'-bis(phenanthren-9-yl)-N,N'-bis(phenyl)benzidine (PAPB), 2,7-bis[N,N-bis(9,9-spirobifluoren-2-yl)amino]-9,9-spirobifluorene (Spiro-5), 2,2'-bis[N,N-bis(biphenyl-4-yl)amino]-9,9-spirobifluorene (2,2'-Spiro-DBP), 2,2'-bis(N,N-diphenylamino)-9.9-spirobifluorene (Spiro-BPA), 2,2',7,7'-tetra(N,N-ditolyl)aminospirobifluorene (Spiro-TTB), N,N,N',N'-tetranaphthalen-2-ylbenzidine (TNB), porphyrin compounds and phthalocyanines such as copper phthalocyanines and titanium oxide phthalocyanines. Hole-transporting polymers typically used are selected from the group consisting of polyvinylcarbazoles, (phenylmethyl)polysilanes and polyanilines. It is likewise possible to obtain hole-transporting polymers by doping hole-transporting molecules into polymers such as polystyrene and polycarbonate. Suitable hole-transporting molecules are the molecules already mentioned above.

In addition - in one embodiment - it is possible to use carbene complexes as hole transport materials, the band gap of the at least one hole transport material generally being greater than the band gap of the emitter material used. In the context of the present application, "band gap" is understood to mean the triplet energy. Suitable carbene complexes are, for example, carbene complexes as described in WO 2005/019373 A2, WO 2006/056418 A2, WO 2005/113704, WO 2007/115970, WO 2007/115981 and WO 2008/000727. One example of a suitable carbene complex is fac-Iridium-tris(1,3-diphenylbenzimidazolin-2-yliden-C,C^{2'}) (Ir(dpbic)₃) with the formula: 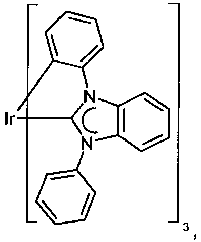 which is disclosed, for example, in WO2005/019373. Preferably, the hole transport layer comprises a compound of formula 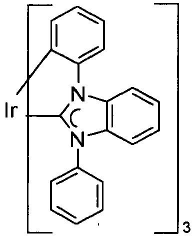 doped with molybdenum oxide (MoOₓ), especially MoO₃, or rhenium oxide (ReOₓ), especially ReO₃. The dopant is contained in an amount of from 0.1 % by weight, preferably 1 to 50 % by weight, more preferably 3 to 15 % by weight, based on the amount of dopant and carbene complex.
The light-emitting layer (3) comprises at least one emitter material. In principle, it may be a fluorescence or phosphorescence emitter, suitable emitter materials being known to those skilled in the art. The at least one emitter material is preferably a phosphorescence emitter. The phosphorescence emitter compounds used with preference are based on metal complexes, and especially the complexes of the metals Ru, Rh, Ir, Pd and Pt, in particular the complexes of Ir, have gained significance.

Suitable metal complexes for use in the inventive OLEDs are described, for example, in documents WO02/60910A1, US 2001/0015432 A1, US 2001/0019782 A1, US2002/0055014A1, US2002/0024293A1, US2002/0048689A1, EP1191612A2, EP 1191613A2, E P 1 211 257 A2, US 2002/0094453 A1, WO 02/02714 A2, WO 00/70655 A2, WO 01/41512 A1, WO 02/15645 A1, WO 2005/019373 A2, WO2005/113704A2, WO 2006/115301 A1, WO 2006/067074 A1, WO 2006/056418, WO 2006121811A1, WO 2007095118 A2, WO 2007/115970, WO 2007/115981, WO 2008/000727, WO2010129323, WO2010056669 and WO10086089.

The light emitting layer comprises preferably a compound of the formula 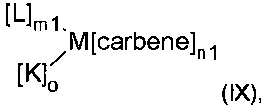 which are described in WO 2005/019373 A2, wherein the symbols have the following meanings:
M is a metal atom selected from the group consisting of Co, Rh, Ir, Nb, Pd, Pt, Fe, Ru, Os, Cr, Mo, W, Mn, Tc, Re, Cu, Ag and Au in any oxidation state possible for the respective metal atom;
Carbene is a carbene ligand which may be uncharged or monoanionic and monodentate, bidentate or tridentate, with the carbene ligand also being able to be a biscarbene or triscarbene ligand;
L is a monoanionic or dianionic ligand, which may be monodentate or bidentate;
K is an uncharged monodentate or bidentate ligand selected from the group consisting of phosphines; phosphonates and derivatives thereof, arsenates and derivatives thereof; phosphites; CO; pyridines; nitriles and conjugated dienes which form a π complex with M¹; n1 is the number of carbene ligands, where n1 is at least 1 and when n1 > 1 the carbene ligands in the complex of the formula I can be identical or different;
m1 is the number of ligands L, where m1 can be 0 or ≥ 1 and when m1 > 1 the ligands L can be identical or different;
o is the number of ligands K, where o can be 0 or ≥ 1 and when o > 1 the ligands K can be identical or different;
   where the sum n1 + m1 + o is dependent on the oxidation state and coordination number of the metal atom and on the denticity of the ligands carbene, L and K and also on the charge on the ligands, carbene and L, with the proviso that n1 is at least 1.

More preferred are metal-carbene complexes of the general formula 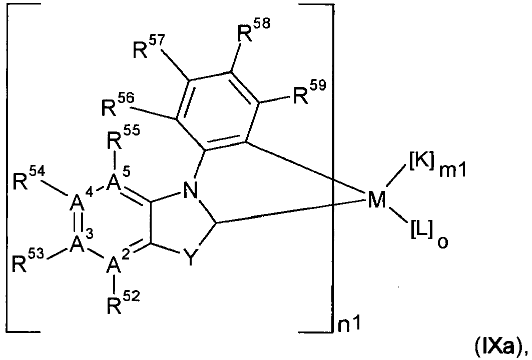 which are described in U.S. patent applications no. 61/286046, 61/323885 and Europen patent application 10187176.2 (WO2011073149), where M, n1, Y, A², A3, A4, A⁵, R⁵¹, R⁵², R⁵³, R⁵⁴, R⁵⁵, R⁵⁶, R⁵⁷, R⁵⁸, R⁵⁹, K, L, m1 and o are each defined as follows:
M is Ir, or Pt,
n1 is an integer selected from 1, 2 and 3,
Y is NR⁵¹, O, S or C(R²⁵)₂,
A², A³, A⁴, and A⁵ are each independently N or C, where 2 A = nitrogen atoms and at least one carbon atom is present between two nitrogen atoms in the ring,
R⁵¹ is a linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms,
R⁵², R⁵³, R⁵⁴ and R⁵⁵ are each, if A², A3, A4 and/or A⁵ is N, a free electron pair, or, if A², A³, A⁴ and/or A⁵ is C, each independently hydrogen, linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms, group with donor or acceptor action, or
R53 and R⁵⁴ together with A³ and A⁴ form an optionally substituted, unsaturated ring optionally interrupted by at least one further heteroatom and having a total of 5 to 18 carbon atoms and/or heteroatoms,
R⁵⁶, R⁵⁷, R⁵⁸ and R⁵⁹ are each independently hydrogen, linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, cycloheteroalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms, group with donor or acceptor action, or
R56 and R⁵⁷, R⁵⁷ and R⁵⁸ or R⁵⁸ and R⁵⁹, together with the carbon atoms to which they are bonded, form a saturated, unsaturated or aromatic, optionally substituted ring optionally interrupted by at least one heteroatom and having a total of 5 to 18 carbon atoms and/or heteroatoms, and/or
if A⁵ is C, R⁵⁵ and R⁵⁶ together form a saturated or unsaturated, linear or branched bridge optionally comprising heteroatoms, an aromatic unit, heteroaromatic unit and/or functional groups and having a total of 1 to 30 carbon atoms and/or heteroatoms, to which is optionally fused a substituted or unsubstituted, five- to eight-membered ring comprising carbon atoms and/or heteroatoms,
R²⁵ is independently a linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms,
K is an uncharged mono- or bidentate ligand,
L is a mono- or dianionic ligand, preferably monoanionic ligand, which may be mono- or bidentate,
m1 is 0, 1 or 2, where, when m1 is 2, the K ligands may be the same or different,
o is 0, 1 or 2, where, when o is 2, the L ligands may be the same or different.

The compound of formula IX is preferably a compound of the formula:

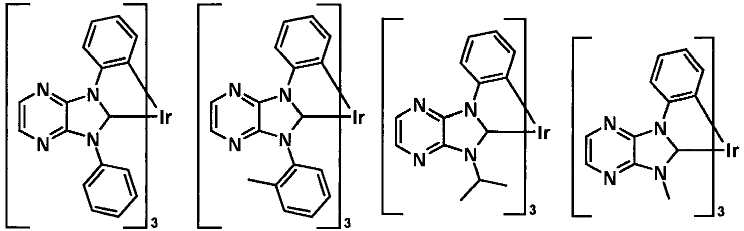

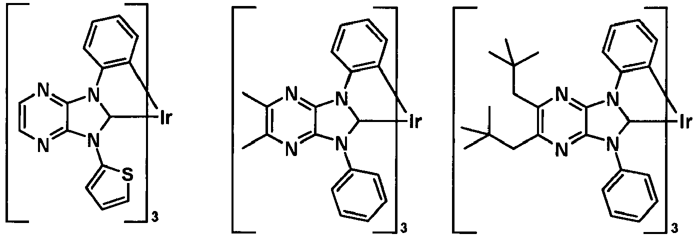

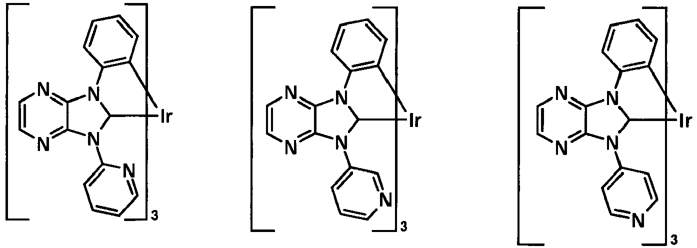

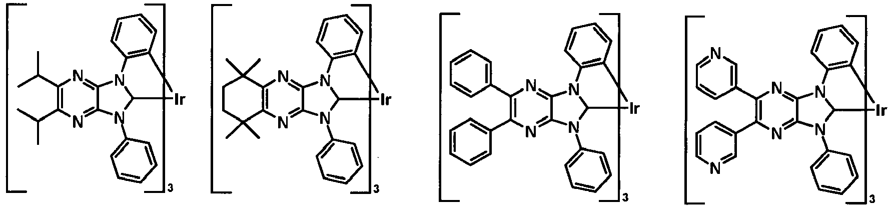

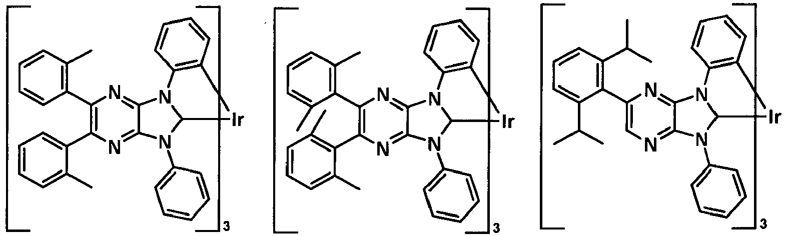

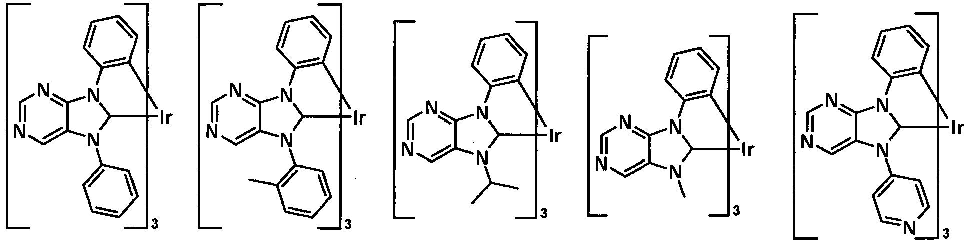

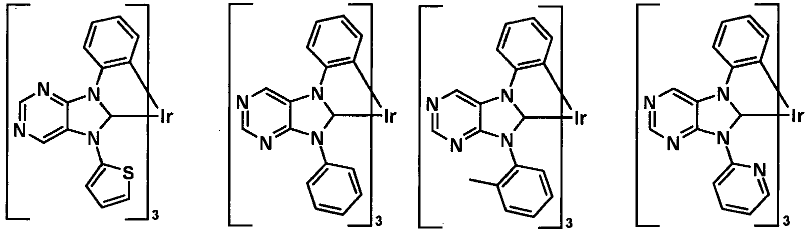

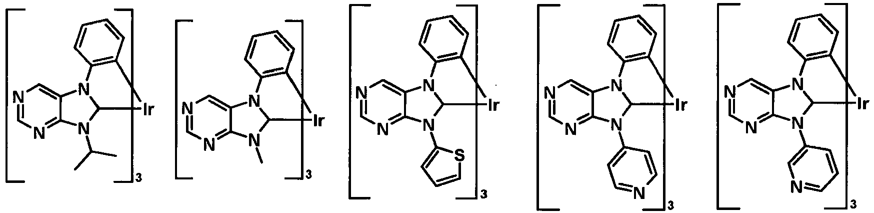

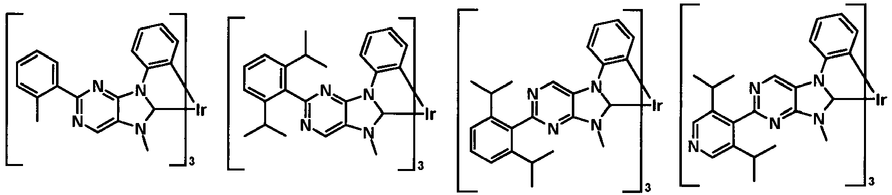

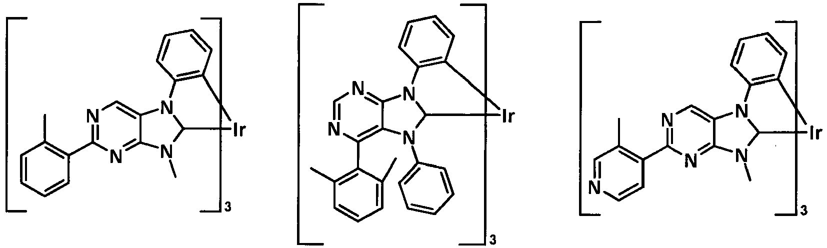

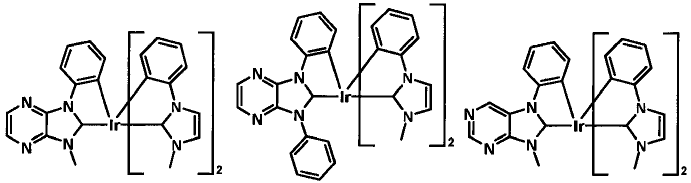

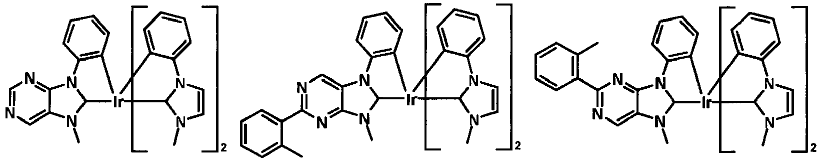

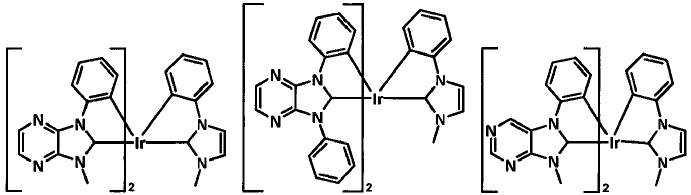

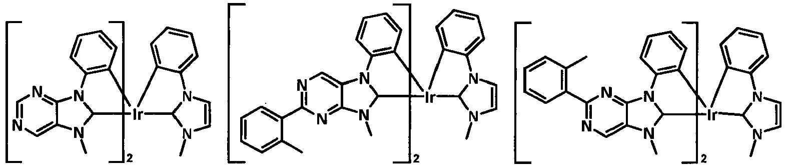

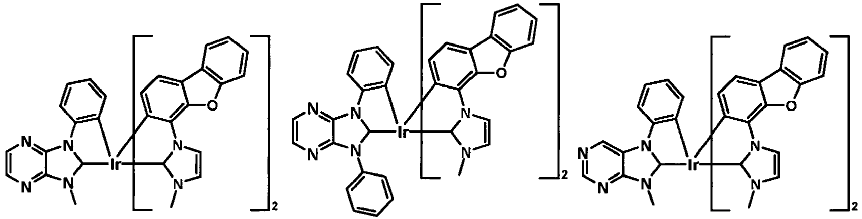

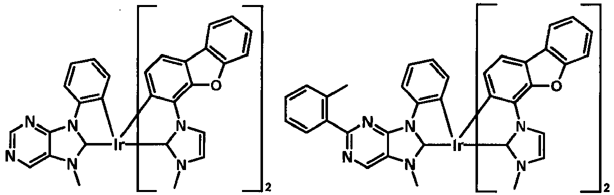

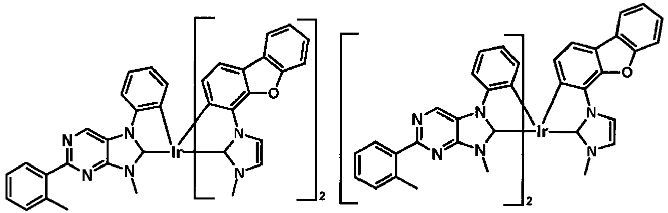

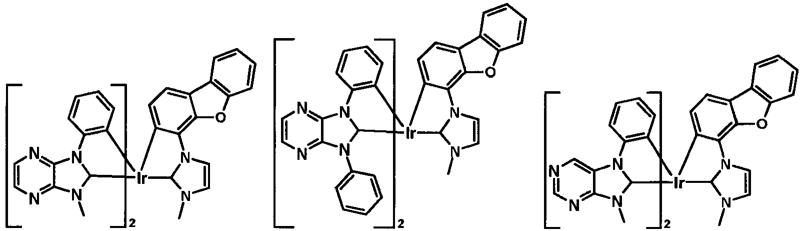

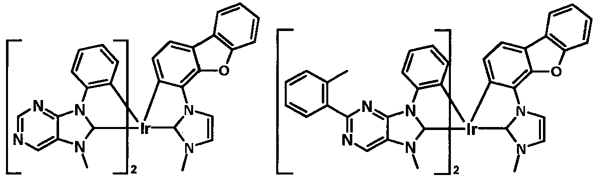

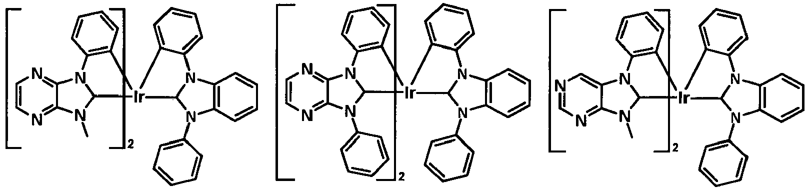

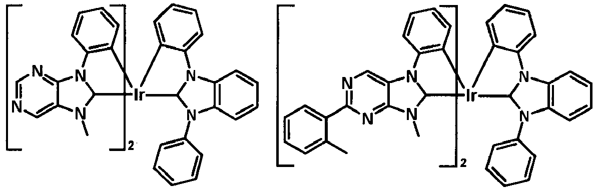

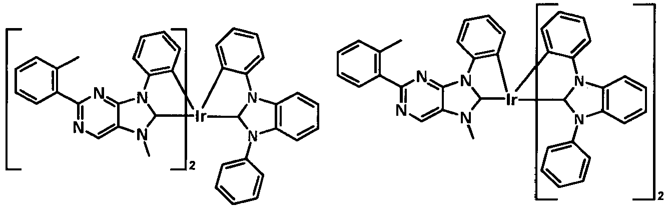

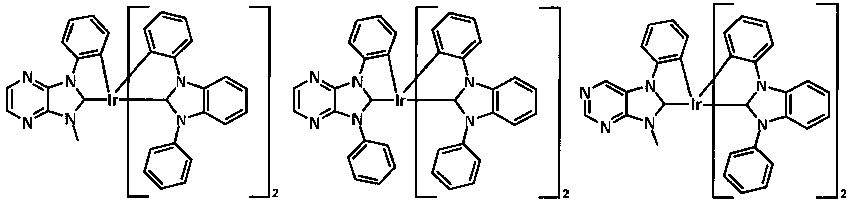

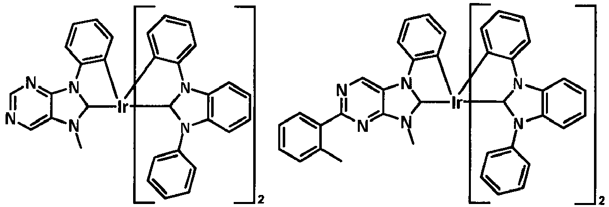

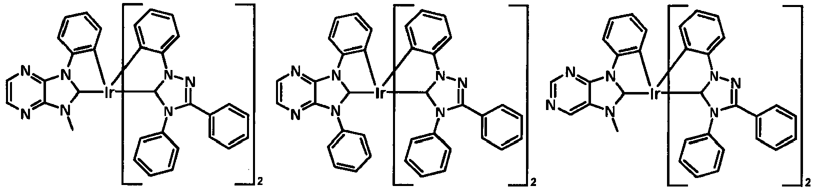

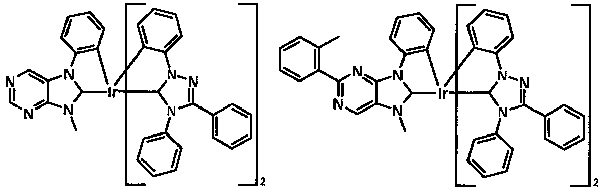

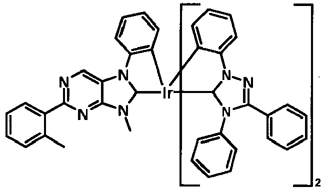

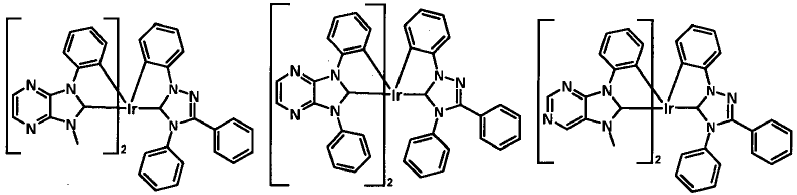

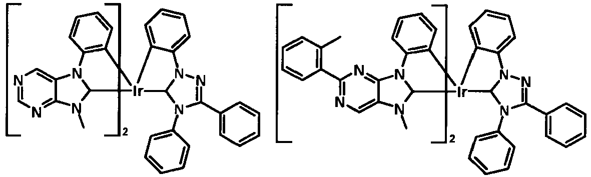

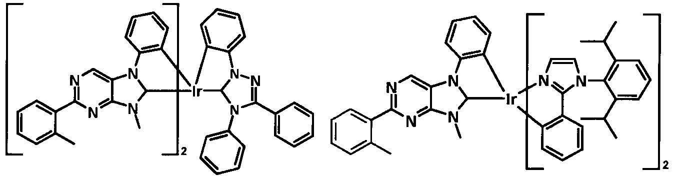

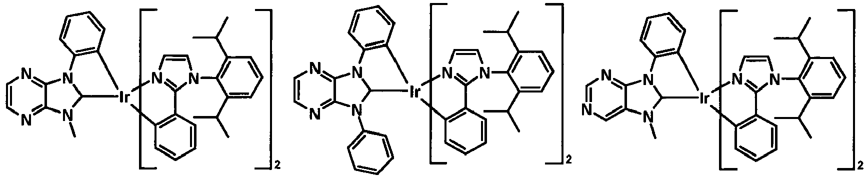

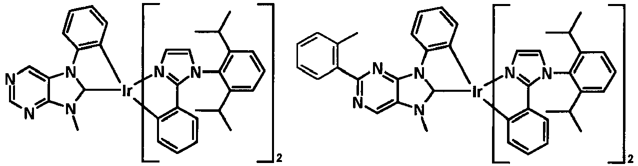

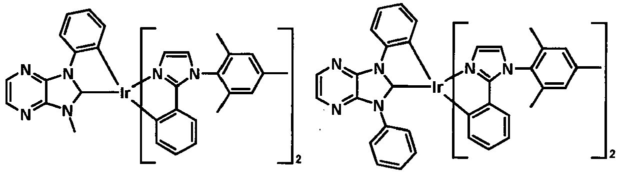

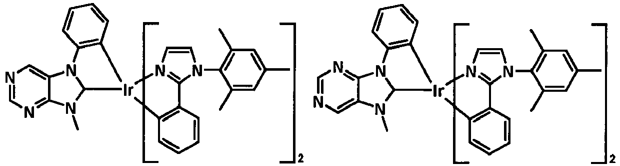

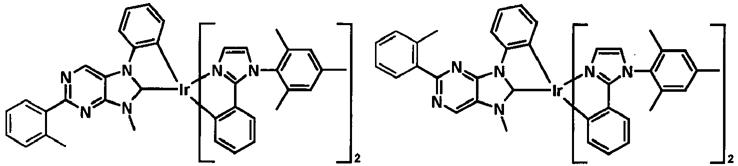

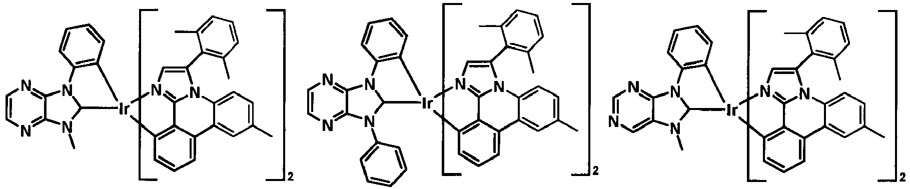

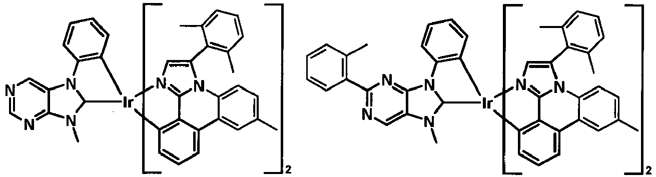

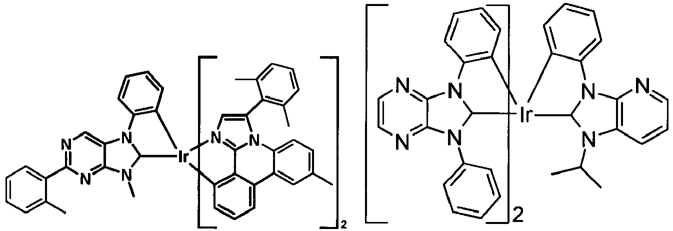

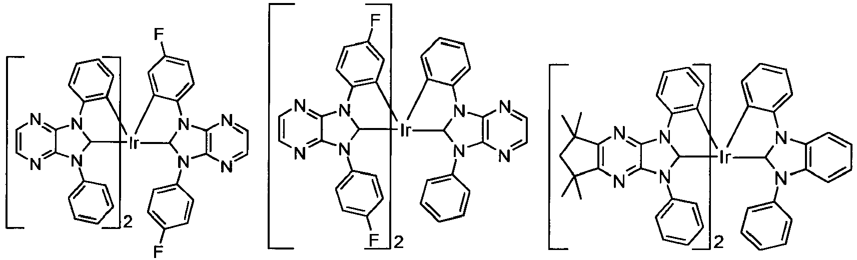

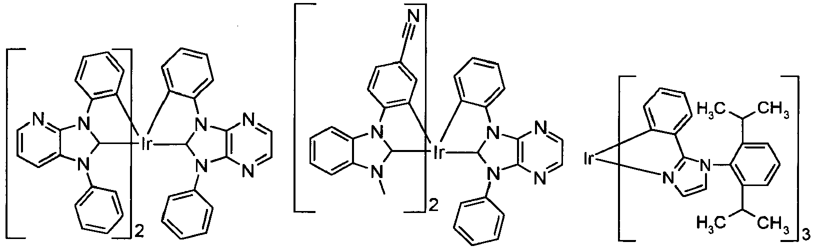

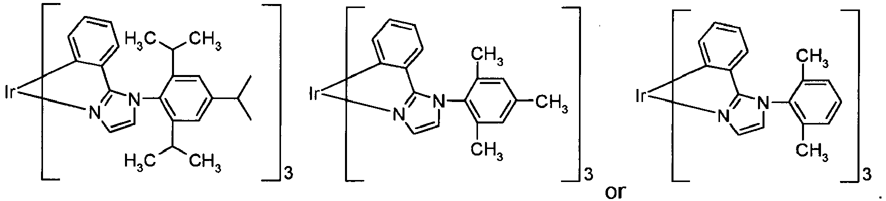

The homoleptic metal-carbene complexes may be present in the form of facial or meridional isomers, preference being given to the facial isomers.

In the case of the heteroleptic metal-carbene complexes, four different isomers may be present, preference being given to the pseudo-facial isomers.

Further suitable metal complexes are the commercially available metal complexes tris(2-phenylpyridine)iridium(III), iridium(III) tris(2-(4-tolyl)pyridinato-N,C^{2'}), b i s (2-phenylpyridine)(acetylacetonato)iridium(III), iridium(III) tris(1-phenylisoquinoline), iridium(III) bis(2,2'-benzothienyl)pyridinato-N,C^{3'})(acetylacetonate), tris(2-phenylquinoline)iridium(III), iridium(III) bis(2-(4,6-difluorophenyl)pyridinato-N,C²)picolinate, iridium(III) bis(1-phenylisoquinoline)(acetylacetonate), bis(2-phenylquinoline)(acetylacetonato)iridium(III), iridium(III) bis(di-benzo[f,h]quinoxaline)(acetylacetonate), iridium(III) bis(2-methyldibenzo[f,h]quinoxaline)(acetylacetonate) and tris(3-methyl-1-phenyl-4-trimethylacetyl-5-pyrazolino)terbium(III), bis[1-(9,9-dimethyl-9H-fluoren-2-yl)isoquinoline](acetyl-acetonato)iridium(III), bis(2-phenylbenzothiazolato)(acetylacetonato)iridium(III), bis(2-(9,9-dihexylfluorenyl)-1-pyridine)(acetylacetonato)iridium(III), bis(2-benzo[b]thiophen-2-yl-pyridine)(acetylacetonato)iridium(III).

In addition, the following commercially available materials are suitable: tris(dibenzoylacetonato)mono(phenanthroline)europium(III), tris(dibenzoylmethane)-mono(phenanthroline)europium(III), tris(dibenzoylmethane)mono(5-aminophenanthroline)-europium(III), tris(di-2-naphthoylmethane)mono(phenanthroline)europium(III), tris(4-bromobenzoylmethane)mono(phenanthroline)europium(III), tris(di(biphenyl)methane)-mono(phenanthroline)europium(III), tris(dibenzoylmethane)mono(4,7-diphenyl-phenanthroline)europium(III), tris(dibenzoylmethane)mono(4,7-di-methyl-phenanthroline)europium(III), tris(dibenzoyl methane) mono (4,7-dimethylphenan-throlinedisulfonic acid)europium(III) disodium salt, tris[di(4-(2-(2-ethoxyethoxy)ethoxy)-benzoylmethane)]mono(phenanthroline)europium(III) and tris[di[4-(2-(2-ethoxy-ethoxy)ethoxy)benzoylmethane)]mono(5-aminophenanthroline)europium(III), osmium(II) bis(3-(trifluoromethyl)-5-(4-tert-butylpyridyl)-1,2,4-triazolato)diphenylmethylphosphine, osmium(II) bis(3-(trifluoromethyl)-5-(2-pyridyl)-1,2,4-triazole)dimethylphenylphosphine, osmium(II) bis(3-(trifluoromethyl)-5-(4-tert-butylpyridyl)-1,2,4-triazolato)dimethylphenylphosphine, osmium(II) bis(3-(trifluoromethyl)-5-(2-pyridyl)-pyrazolato)dimethylphenylphosphine, tris[4,4'-di-tert-butyl(2,2')-bipyridine]ruthenium(III), osmium(II) bis(2-(9,9-dibutylfluorenyl)-1-isoquinoline(acetylacetonate).

Suitable triplet emitters are, for example, carbene complexes. In one embodiment of the present invention, the compounds of the formula X are used in the light-emitting layer as matrix material together with carbene complexes as triplet emitters. 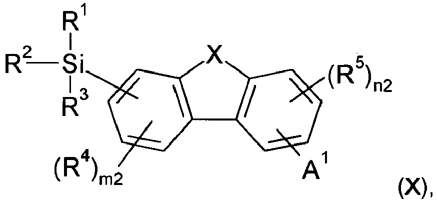 wherein X is NR, S, O or PR;
R is aryl, heteroaryl, alkyl, cycloalkyl, or heterocycloalkyl;
A¹ is -NR⁶R⁷, -P(O)R⁸R⁹, -PR¹⁰R¹¹, -S(O)₂R¹², -S(O)R¹³ -SR¹⁴, or -OR¹⁵;
R¹, R² and R³ are independently of each other aryl, heteroaryl, alkyl, cycloalkyl, or heterocycloalkyl, wherein at least on of the groups R¹, R², or R³ is aryl, or heteroaryl;
R⁴ and R⁵ are independently of each other alkyl, cycloalkyl, heterocycloalkyl, aryl, heteroaryl, a group A, or a group having donor, or acceptor characteristics;
n2 and m2 are independently of each other 0, 1, 2, or 3;
R⁶, R⁷ form together with the nitrogen atom a cyclic residue having 3 to 10 ring atoms, which can be unsubstituted, or which can be substituted with one, or more substituents selected from alkyl, cycloalkyl, heterocycloalkyl, aryl, heteroaryl and a group having donor, or acceptor characteristics; and/or which can be annulated with one, or more further cyclic residues having 3 to 10 ring atoms, wherein the annulated residues can be unsubstituted, or can be substituted with one, or more substituents selected from alkyl, cycloalkyl, heterocycloalkyl, aryl, heteroaryl and a group having donor, or acceptor characteristics; and
R⁸, R⁹, R¹⁰, R¹¹, R¹², R¹³, R¹⁴ und R¹⁵ are independently of each other aryl, heteroaryl, alkyl, cycloalkyl, or heterocycloalkyl. Compounds of formula X, such as, for example, 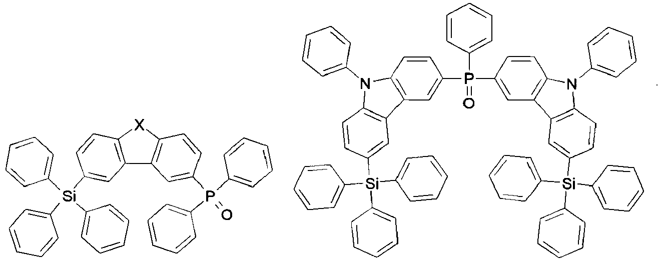 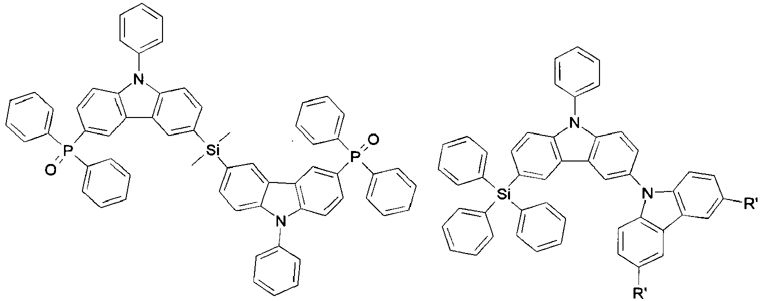 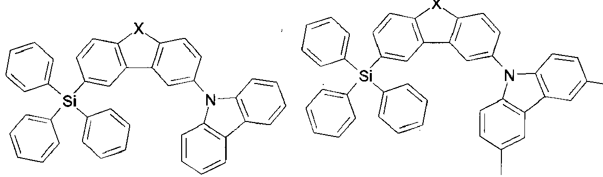 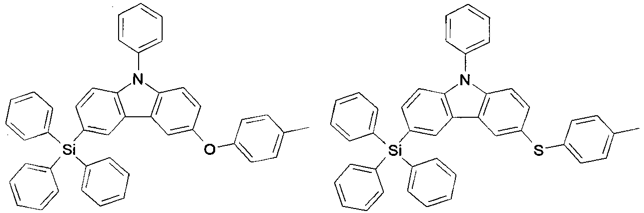 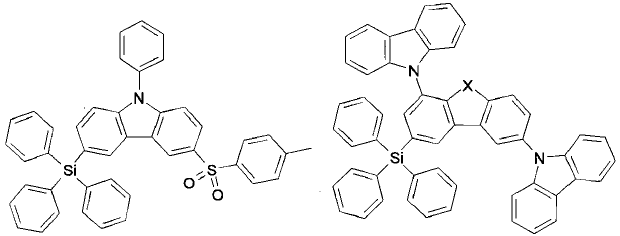 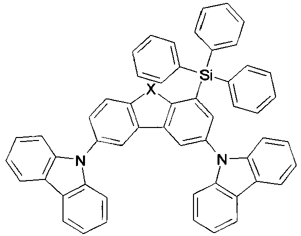 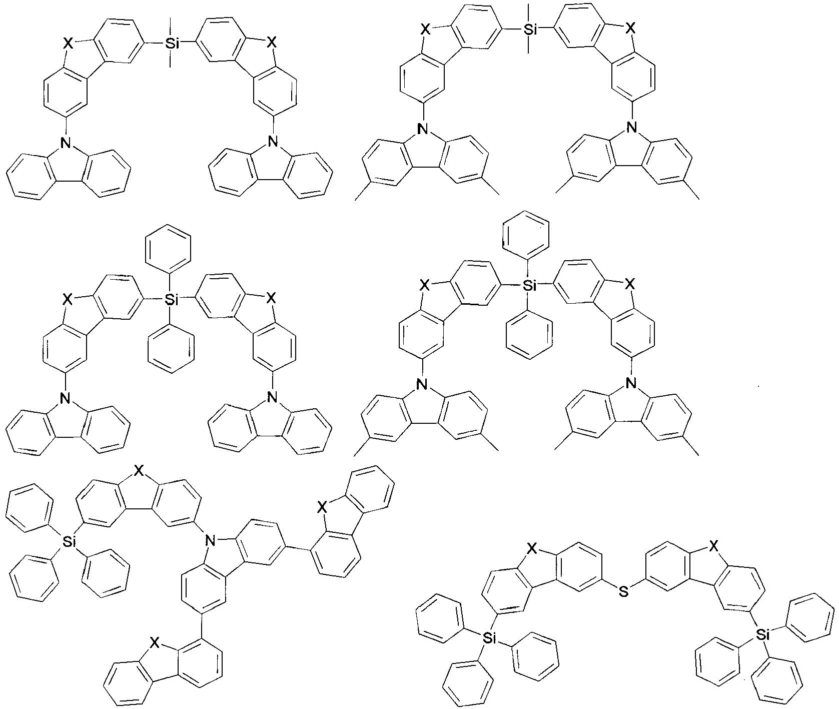 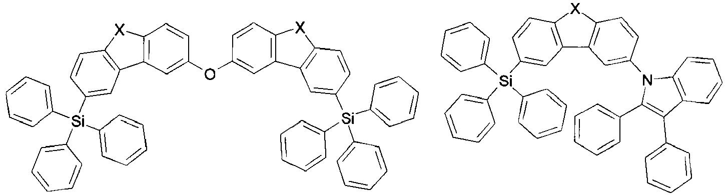 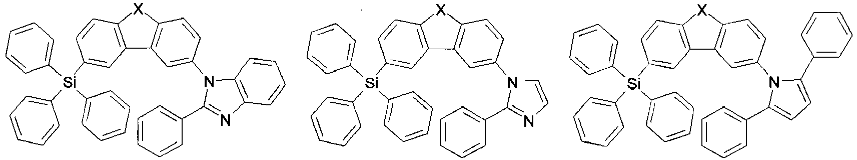 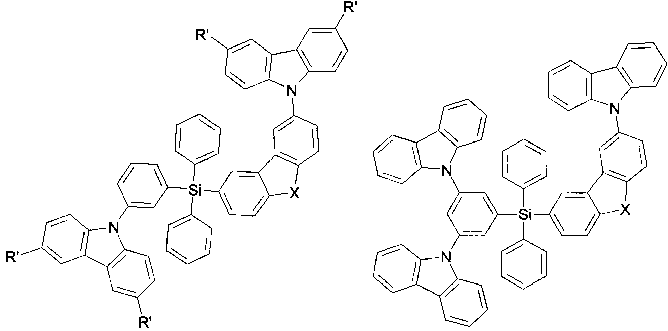 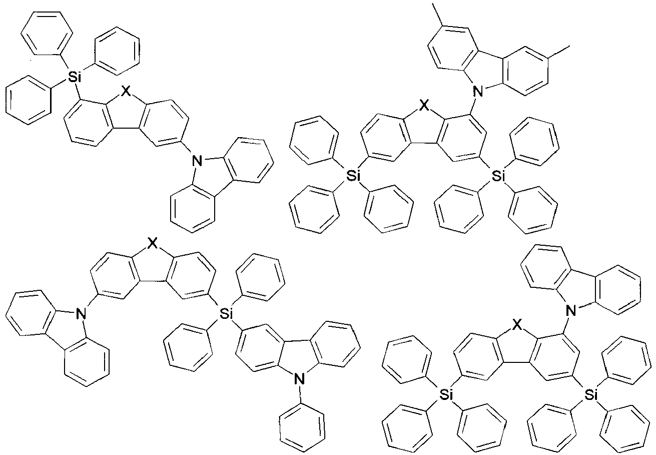 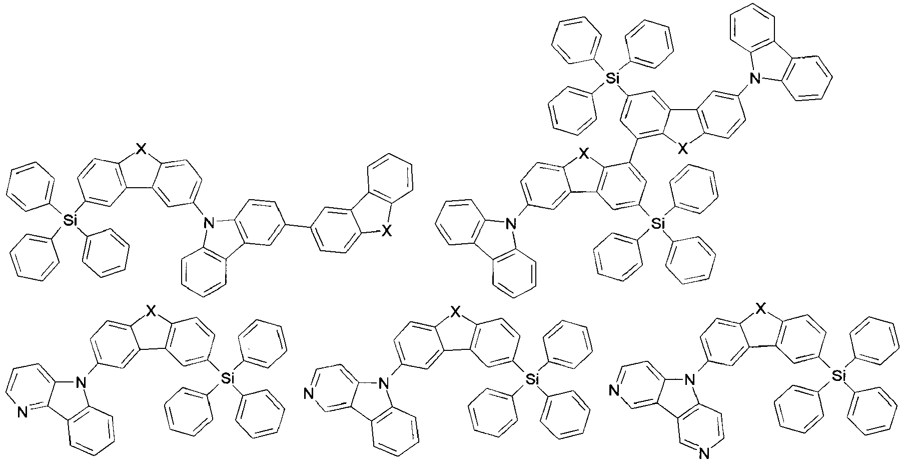 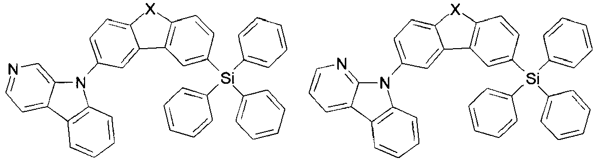 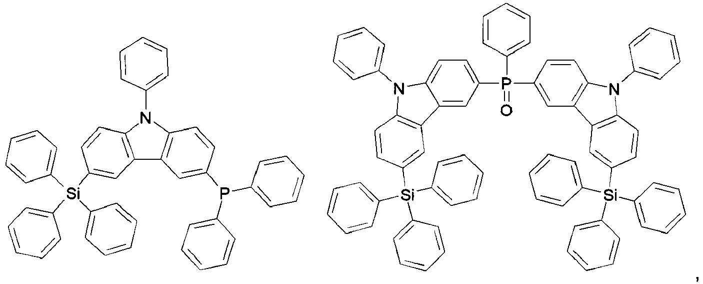 wherein X is S, or O, and R' is H, or CH₃; 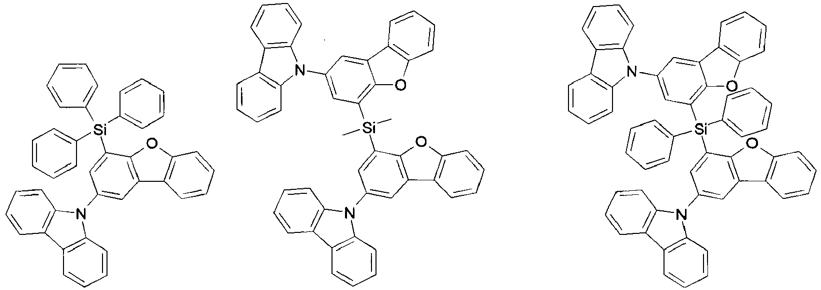 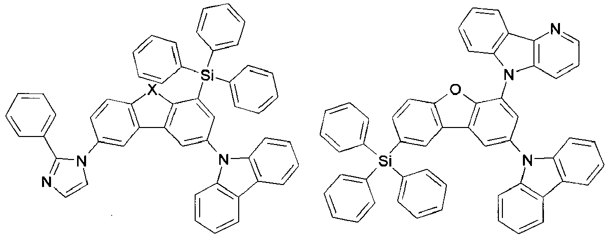 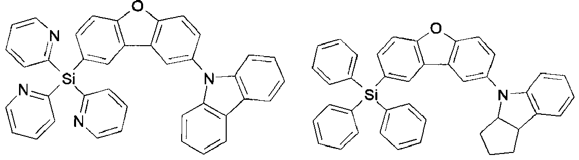 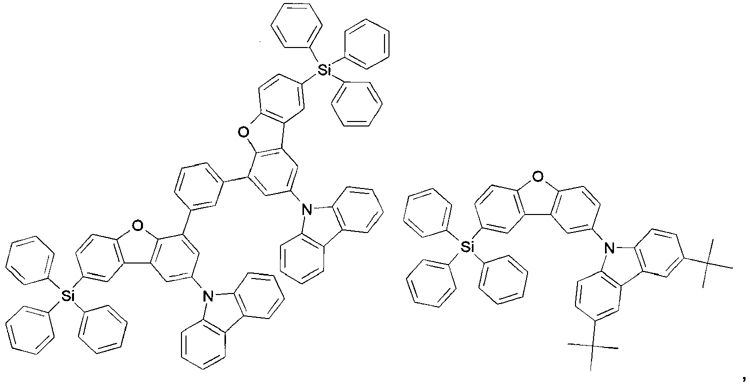 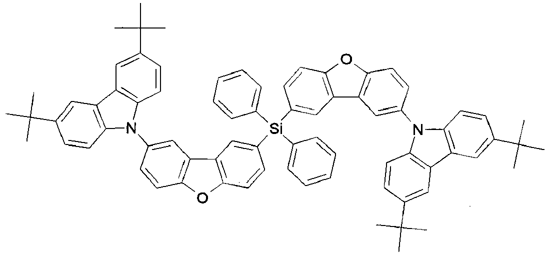 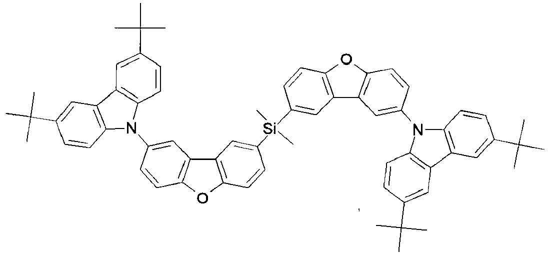 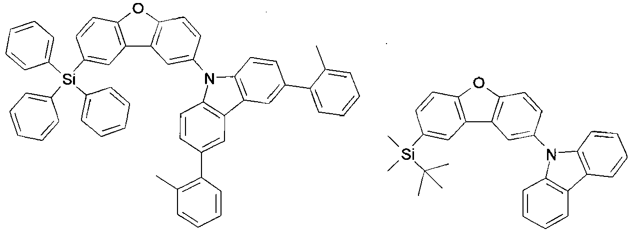 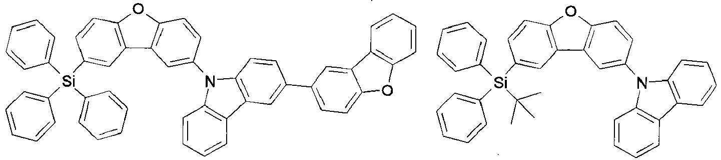 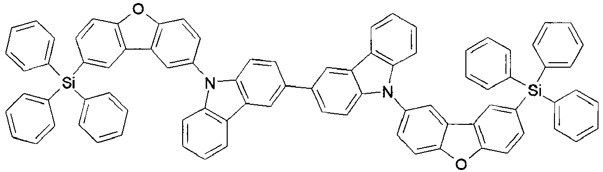 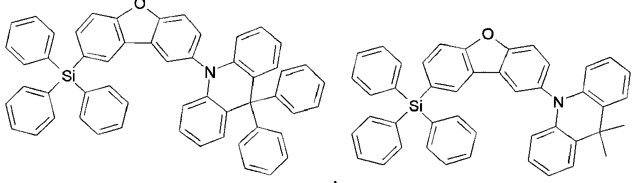 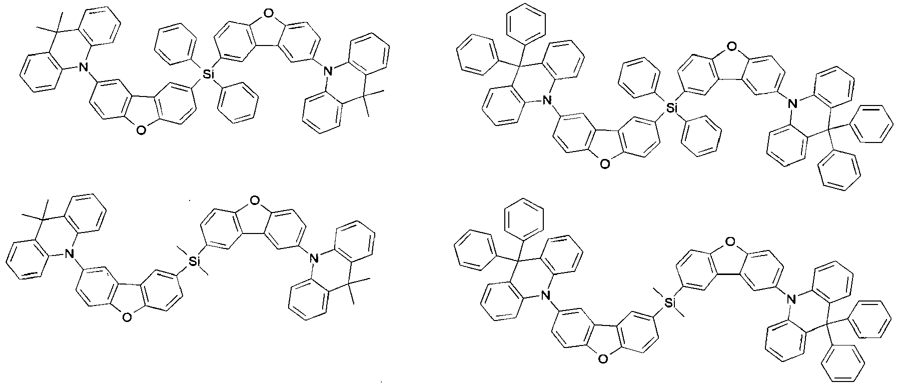 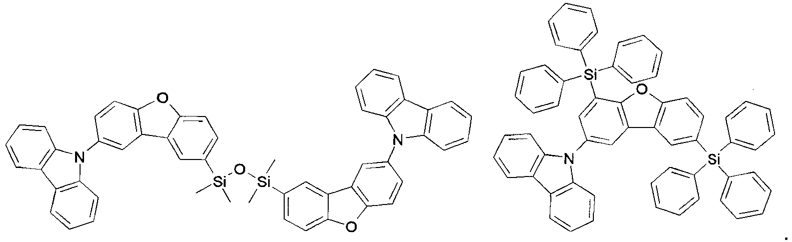 are described in WO2010/079051.

In addition, dibenzofuranes described, for example, in US2009066226, EP1885818B1, EP1970976, EP1998388, EP2034538, US2007/0224446A1, WO2009/069442A1, WO2010/090077A1 und JP 2006/321750A are suitable as matrix materials. Examples of preferred matrix materials are shown below: 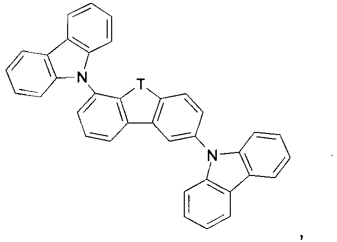 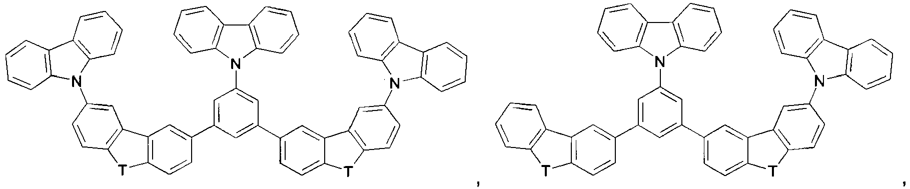 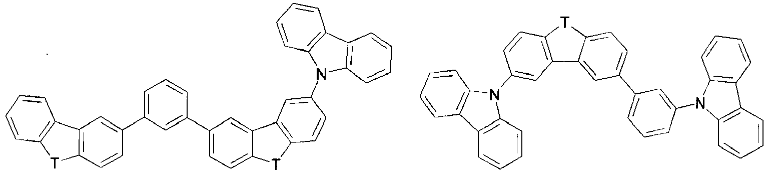 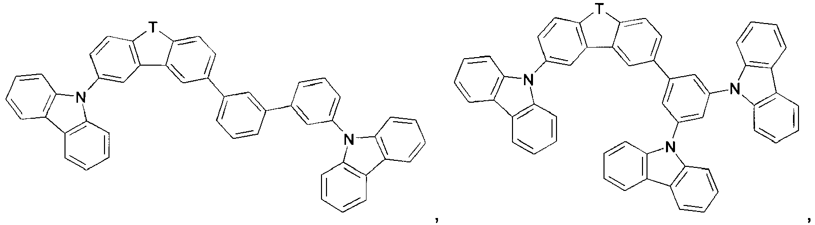 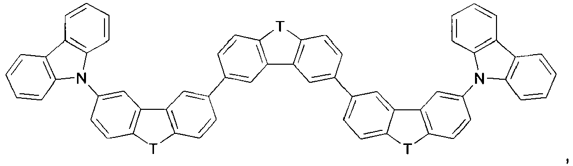 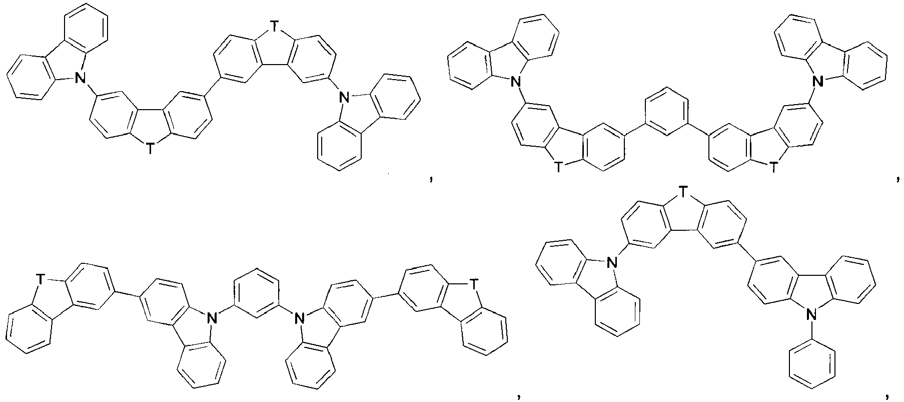 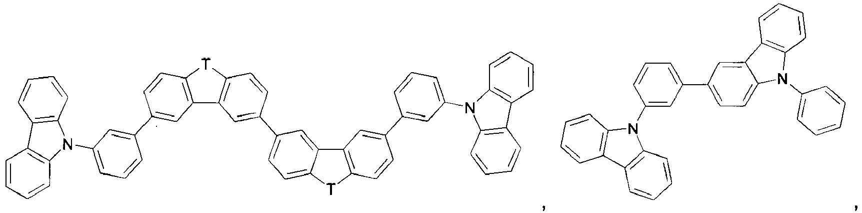 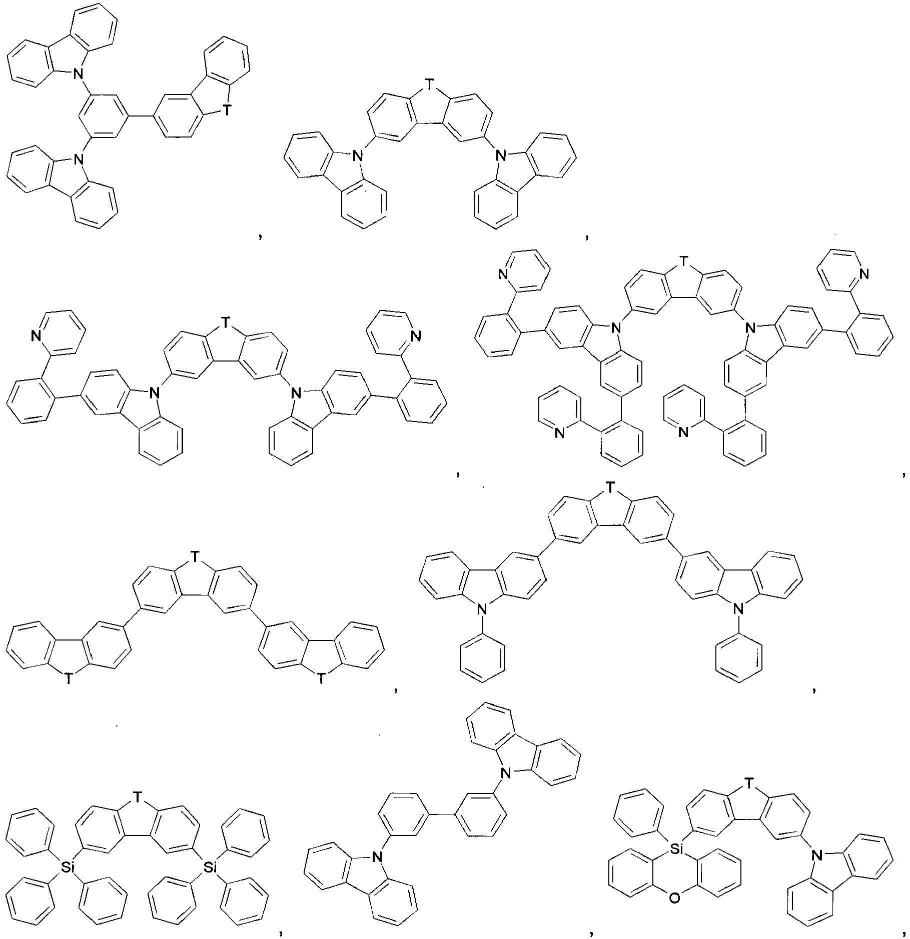 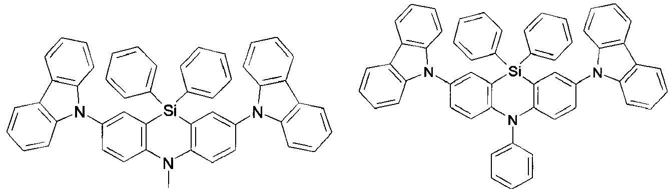 and

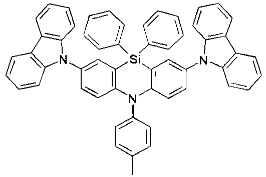

In the above-mentioned compounds T is O, or S, preferably O. If T occurs more than one time in a molecule, all groups T have the same meaning.

Suitable carbene complexes are known to those skilled in the art and are described, for example, in WO 2005/019373 A2, WO 2006/056418 A2, WO 2005/113704, WO 2007/115970, WO 2007/115981 and WO 2008/000727.

The light-emitting layer may comprise further components in addition to the emitter material. For example, a fluroescent dye may be present in the light-emitting layer in order to alter the emission color of the emitter material. In addition - in a preferred embodiment - a matrix material can be used. This matrix material may be a polymer, for example poly(N-vinylcarbazole) or polysilane. The matrix material may, however, be a small molecule, for example 4,4'-N,N'-dicarbazolebiphenyl (CDP=CBP) or tertiary aromatic amines, for example TCTA. In a preferred embodiment of the present invention, at least one compound of the formula X is used as matrix material.

In a preferred embodiment, the light-emitting layer is formed from 2 to 40% by weight, preferably 5 to 35% by weight, of at least one of the aforementioned emitter materials and 60 to 98% by weight, preferably 65 to 95% by weight, of at least one of the aforementioned matrix materials - in one embodiment at least one compound of the formula X - where the sum total of the emitter material and of the matrix material adds up to 100% by weight.

In a preferred embodiment, the light-emitting layer comprises a compound of formula X, such as, for example, 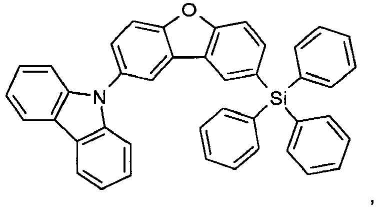 or 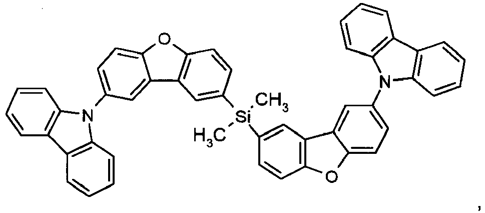 and two carbene complexes, preferably of formula 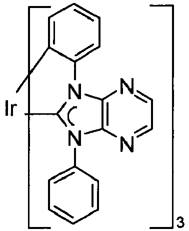 and 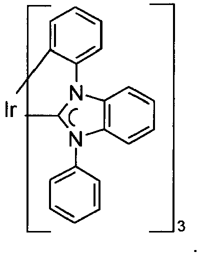 In said embodiment, the light-emitting layer is formed from 2 to 40% by weight, preferably 5 to 35% by weight, of 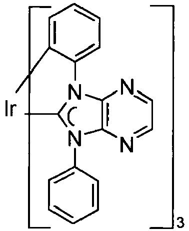 and 60 to 98% by weight, preferably 65 to 95% by weight, of a compound of the formula X and 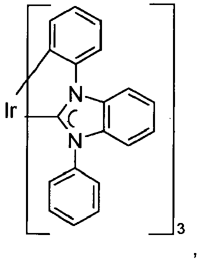 where the sum total of the carben complexes and of the compound of formula X adds up to 100% by weight.

In a further embodiment, the compounds of the formula X are used as hole/exciton blocker material, preferably together with carbene complexes as triplet emitters. The compounds of the formula X may be used as matrix materials or both as matrix materials and as hole/exciton blocker materials together with carbene complexes as triplet emitters.

Suitable metal complexes for use together with the compounds of the formula X as matrix material and/or hole/exciton blocker material, in OLEDs are thus, for example, also carbene complexes as described in WO 2005/019373 A2, WO 2006/056418 A2, WO 2005/113704, WO 2007/115970, WO 2007/115981 and WO 2008/000727.

Hole blocker materials typically used in OLEDs are compounds of formula X, 2,6-bis(N-carbazolyl)pyridine (mCPy), 2,9-dimethyl-4,7-diphenyl-1,10-phenanthroline (bathocuproin, (BCP)), bis(2-methyl-8-quinolinato)-4-phenylphenylato)aluminum(III) (BAlq), phenothiazine S,S-dioxide derivates and 1,3,5-tris(N-phenyl-2-benzylimidazolyl)benzene) (TPBI), TPBI also being suitable as electron-conducting material. Further suitable hole blockers and/or electron transport materials are 2,2',2"-(1,3,5-benzenetriyl)tris(1-phenyl-1-H-benzimidazole), 2-(4-biphenylyl)-5-(4-tert-butylphenyl)-1,3,4-oxadiazole, 8-hydroxyquinolinolatolithium, 4-(naphthalen-1-yl)-3,5-diphenyl-4H-1,2,4-triazole, 1,3-bis[2-(2,2'-bipyridin-6-yl)-1,3,4-oxadiazo-5-yl]benzene, 4,7-diphenyl-1,10-phenanthroline, 3-(4-biphenylyl)-4-phenyl-5-tert-butylphenyl-1,2,4-triazole, 6,6'-bis[5-(biphenyl-4-yl)-1,3,4-oxadiazo-2-yl]-2,2'-bipyridyl, 2-phenyl-9,10-di(naphthalene-2-yl)anthracene, 2,7-bis[2-(2,2'-bipyridin-6-yl)-1,3,4-oxadiazo-5-yl]-9,9-dimethylfluorene, 1,3-bis[2-(4-tert-butylphenyl)-1,3,4-oxadiazo-5-yl]benzene, 2-(naphthalene-2-yl)-4,7-diphenyl-1,10-phenanthroline, tris-(2,4,6-trimethyl-3-(pyridin-3-yl)phenyl)borane, 2,9-bis(naphthalene-2-yl)-4,7-diphenyl-1,10-phenanthroline, 1-methyl-2-(4-(naphthalene-2-yl)phenyl)-1H-imidazo[4,5-f][1,10]-phenanthroline. In a further embodiment, it is possible to use compounds which comprise aromatic or heteroaromatic rings joined via groups comprising carbonyl groups, as disclosed in WO2006/100298, disilyl compounds selected from the group consisting of disilyl-carbazoles, disilylbenzofurans, disilylbenzothiophenes, disilylbenzophospholes, disilylbenzothiophene S-oxides and disilylbenzothiophene S,S-dioxides, as specified, for example, in WO2009003919 (PCT/EP2008/058207) and WO2009003898 (PCT/EP2008/058106) and disilyl compounds as disclosed in WO2008/034758, as a blocking layer for holes/excitons (4) or as matrix materials in the light-emitting layer (3).

In addition - in one embodiment - it is possible to use carbene complexes as hole transport materials, the band gap of the at least one hole transport material generally being greater than the band gap of the emitter material used. In the context of the present application, "band gap" is understood to mean the triplet energy. Suitable carbene complexes are, for example, carbene complexes as described in WO 2005/019373 A2, WO 2006/056418 A2, WO 2005/113704, WO 2007/115970, WO 2007/115981 and WO 2008/000727. One example of a suitable carbene complex is fac-Iridium-tris(1,3-diphenylbenzimidazolin-2-yliden-C,C^{2I}) (Ir(dpbic)₃) with the formula: 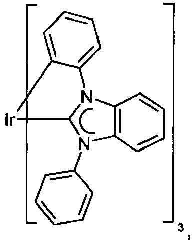 which is disclosed, for example, in WO2005/019373. Preferably, the hole transport layer comprises Ir(dpbic)₃ doped with molybdenum oxide (MoOₓ), especially MoO₃, or rhenium oxide (ReOₓ), especially ReO₃. The dopant is contained in an amount of from 0.1 % by weight, preferably 1 to 8 % by weight, more preferably 3 to 5 % by weight, based on the amount of dopant and carbene complex.

In a preferred embodiment, the present invention relates to an inventive OLED comprising the layers (1) anode, (2) hole transport layer, (3) light-emitting layer, (4) blocking layer for holes/excitons, (5) electron transport layer and (6) cathode, and if appropriate further layers, wherein the electron transport layer comprises the compounds of formula I, or II.

The electron transport layer (5) of the inventive OLEDs comprises the compounds of formula I, or II. The layer (5) preferably improves the mobility of the electrons.

Among the materials mentioned above as hole transport materials and electron transport materials, some may fulfil several functions. For example, some of the electron-transporting materials are simultaneously hole-blocking materials when they have a low-lying HOMO. These can be used, for example, in the blocking layer for holes/excitons (4).

The charge transport layers can also be electronically doped in order to improve the transport properties of the materials used, in order firstly to make the layer thicknesses more generous (avoidance of pinholes/short circuits) and in order secondly to minimize the operating voltage of the device. For example, the hole transport materials can be doped with electron acceptors; for example, phthalocyanines or arylamines such as TPD or TDTA can be doped with tetrafluorotetracyanquinodimethane (F4-TCNQ) or with MoO₃ or WO₃. The electron transport materials can be doped, for example, with alkali metals, for example Alq₃ with lithium. In addition, electron transports can be doped with salts such as Cs₂CO₃, or 8-hydroxyquinolato-lithium (Liq). Electronic doping is known to those skilled in the art and is disclosed, for example, in W. Gao, A. Kahn, J. Appl. Phys., Vol. 94, No. 1, 1 July 2003 (p-doped organic layers); A. G. Werner, F. Li, K. Harada, M. Pfeiffer, T. Fritz, K. Leo. Appl. Phys. Lett., Vol. 82, No. 25, 23 June 2003 and Pfeiffer et al., Organic Electronics 2003, 4, 89 - 103. For example, the hole transport layer may, in addition to a carbene complex, e.g. Ir(dpbic)₃, be doped with MoO₃ or WO₃.

The cathode (6) is an electrode which serves to introduce electrons or negative charge carriers. Suitable materials for the cathode are selected from the group consisting of alkali metals of group Ia, for example Li, Cs, alkaline earth metals of group IIa, for example calcium, barium or magnesium, metals of group IIb of the periodic table of the elements (old IUPAC version), comprising the lanthanides and actinides, for example samarium. In addition, it is also possible to use metals such as aluminum or indium, and combinations of all metals mentioned. In addition, alkali metal-comprising organometallic compounds, or alkali metal fluorides, such as, for example, LiF, CsF, or KF, can be applied between the organic layer and the cathode in order to reduce the operating voltage.

The OLED according to the present invention may additionally comprise further layers which are known to those skilled in the art. For example, a layer which facilitates the transport of the positive charge and/or matches the band gaps of the layers to one another may be applied between the layer (2) and the light-emitting layer (3). Alternatively, this further layer may serve as a protective layer. In an analogous manner, additional layers may be present between the light-emitting layer (3) and the layer (4) in order to facilitate the transport of negative charge and/or to match the band gaps between the layers to one another. Alternatively, this layer may serve as a protective layer.

In a preferred embodiment, the inventive OLED, in addition to layers (1) to (6), comprises at least one of the following layers mentioned below:
- a hole injection layer between the anode (1) and the hole-transport layer (2);
- a blocking layer for electrons between the hole-transport layer (2) and the light-emitting layer (3);
- an electron injection layer between the electron-transport layer (5) and the cathode (6).

Materials for a hole injection layer may be selected from copper phthalocyanine, 4,4',4"-tris(N-3-methylphenyl-N-phenylamino)triphenylamine (m-MTDATA), 4,4',4"-tris(N-(2-naphthyl)-N-phenylamino)triphenylamine (2T-NATA), 4,4',4"-tris(N-(1-naphthyl)-N-phenylamino)triphenylamine (1T-NATA), 4,4',4"-tris(N,N-diphenylamino)triphenylamine (NATA), titanium oxide phthalocyanine, 2,3,5,6-tetrafluoro-7,7,8,8-tetracyanoquino-dimethane (F4-TCNQ), pyrazino[2,3-f][1,10]phenanthroline-2,3-dicarbonitrile (PPDN), N,N,N',N'-tetrakis(4-methoxyphenyl)benzidine (MeO-TPD), 2,7-bis[N,N-bis(4-methoxyphenyl)amino]-9,9-spirobifluorene (MeO-Spiro-TPD), 2,2'-bis[N,N-bis(4-methoxy-phenyl)amino]-9,9-spirobifluorene (2,2'-MeO-Spiro-TPD), N,N'-diphenyl-N,N'-di-[4-(N,N-ditolylamino)phenyl]benzidine (NTNPB), N,N'-diphenyl-N,N'-di-[4-(N,N-diphenyl-amino)phenyl]benzidine (NPNPB), N,N'-di(naphthalen-2-yl)-N,N'-diphenylbenzene-1,4-diamine (α-NPP). In principle, it is possible that the hole injection layer comprises at least one compound of the formula X as hole injection material.

As a material for the electron injection layer, CsF, KF, or lithium quinolate (Liq), for example, can be selected. CsF is more preferred than KF, or Liq.

The person skilled in the art is aware (for example on the basis of electrochemical studies) of how suitable materials have to be selected. Suitable materials for the individual layers are known to those skilled in the art and are disclosed, for example, in WO 00/70655.

In addition, it is possible that some of the layers used in the inventive OLED have been surface-treated in order to increase the efficiency of charge carrier transport. The selection of the materials for each of the layers mentioned is preferably determined by obtaining an OLED with a high efficiency and lifetime.

The inventive OLED can be produced by methods known to those skilled in the art. In general, the inventive OLED is produced by successive vapor deposition of the individual layers onto a suitable substrate. Suitable substrates are, for example, glass, inorganic semi-transports, typically ITO, or IZO, or polymer films. For vapor deposition, it is possible to use customary techniques, such as thermal evaporation, chemical vapor deposition (CVD), physical vapor deposition (PVD) and others. In an alternative process, the organic layers of the OLED can be applied from solutions or dispersions in suitable solvents, employing coating techniques known to those skilled in the art.

In general, the different layers have the following thicknesses: anode (1) 50 to 500 nm, preferably 100 to 200 nm; hole-conducting layer (2) 5 to 100 nm, preferably 20 to 80 nm, light-emitting layer (3) 1 to 100 nm, preferably 10 to 80 nm, blocking layer for holes/excitons (4) 2 to 100 nm, preferably 5 to 50 nm, electron-conducting layer (5) 5 to 100 nm, preferably 20 to 80 nm, cathode (6) 20 to 1000 nm, preferably 30 to 500 nm. The relative position of the recombination zone of holes and electrons in the inventive OLED in relation to the cathode and hence the emission spectrum of the OLED can be influenced, among other factors, by the relative thickness of each layer. This means that the thickness of the electron transport layer should preferably be selected such that the position of the recombination zone is matched to the optical resonator property of the diode and hence to the emission wavelength of the emitter. The ratio of the layer thicknesses of the individual layers in the OLED depends on the materials used. The layer thicknesses of any additional layers used are known to those skilled in the art. It is possible that the electron-conducting layer and/or the hole-conducting layer have greater thicknesses than the layer thicknesses specified when they are electrically doped.

Use of the electron transport layer of the present application makes it possible to obtain OLEDs with high efficiency and with low operating voltage. Frequently, the OLEDs obtained by the use of the electron transport layer of the present application additionally have high lifetimes. The efficiency of the OLEDs can additionally be improved by optimizing the other layers of the OLEDs. Shaped substrates and novel hole-transport materials which bring about a reduction in the operating voltage or an increase in the quantum efficiency are likewise usable in the inventive OLEDs. Moreover, additional layers may be present in the OLEDs in order to adjust the energy level of the different layers and to facilitate electroluminescence.

The OLEDs may further comprise at least one second light-emitting layer. The overall emission of the OLEDs may be composed of the emission of the at least two light-emitting layers and may also comprise white light.

The OLEDs can be used in all apparatus in which electroluminescence is useful. Suitable devices are preferably selected from stationary and mobile visual display units and illumination units. Stationary visual display units are, for example, visual display units of computers, televisions, visual display units in printers, kitchen appliances and advertising panels, illuminations and information panels. Mobile visual display units are, for example, visual display units in cellphones, laptops, digital cameras, MP3 players, vehicles and destination displays on buses and trains. Further devices in which the inventive OLEDs can be used are, for example, keyboards; items of clothing; furniture; wallpaper.

In addition, the electron transport layer of the present application can be used in OLEDs with inverse structure. The structure of inverse OLEDs and the materials typically used therein are known to those skilled in the art. In addition, the present invention relates to an apparatus selected from the group consisting of stationary visual display units such as visual display units of computers, televisions, visual display units in printers, kitchen appliances and advertising panels, illuminations, information panels, and mobile visual display units such as visual display units in cellphones, laptops, digital cameras, MP3 players, vehicles and destination displays on buses and trains; illumination units; keyboards; items of clothing; furniture; wallpaper, comprising the inventive organic electronic device, or the inventive organic layer, especially electron transport layer.

The following examples are included for illustrative purposes only and do not limit the scope of the claims. Unless otherwise stated, all parts and percentages are by weight.

### Examples

### Example 1

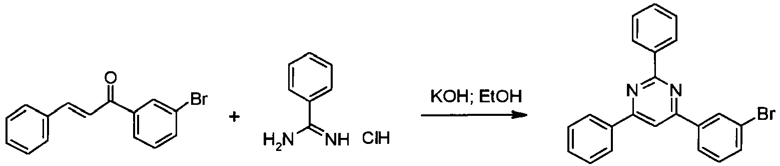

a) 5.45 g (34.8 mmol) benzamidine hydrochloride hydrate and 4.10 g (73.1 mmol) potassium carbonate in 50 ml ethanol are stirred at 90 °C under dry air. 20.0 g (69.7 mmol) (E)-1-(3-bromophenyl)-3-phenyl-prop-2-en-1-one in 20 ml hot ethoxy-ethanol are added slowly. After 24 h the reaction mixture is cooled to 25 °C, the product is filtered off, washed with ethanol, water and again ethanol and is used without further purification in step b) (yield 7.84 g (58 %)). 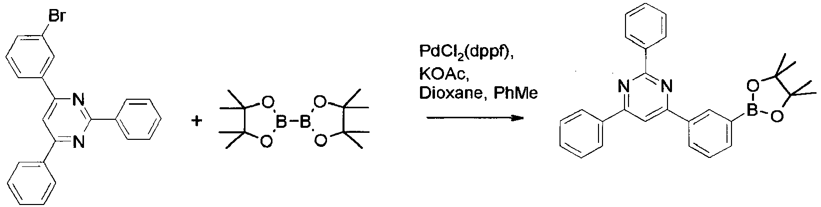
b) 5.20 g (13.4 mmol) 4-(3-bromophenyl)-2,6-diphenyl-pyrimidine, 8.18 g (32.2 mmol) 4,4,5,5-tetramethyl-2-(4,4,5,5-tetramethyl-1,3,2-dioxaborolan-2-yl)-1,3,2-dioxaborolane and 7.91 g (80.6 mmol) potassium acetate are degassed with argon. 60 ml DMF are added and the mixture is degassed with argon. 120 mg (0.16 mmol) PdCl₂(dppf) (1,1'-bis(diphenylphosphino)ferrocene-palladium(II) dichloride) are added. The reaction mixture is stirred at 60 °C for 26 h, poured on ice and the product is filtered off. 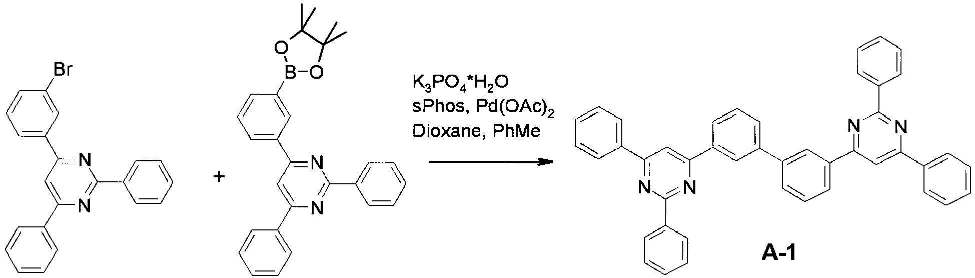
c) 5.55 g (12.8 mmol) of the product of example 1a, 4.50 g (11.6 mmol) 4-(3-bromophenyl)-2,6-diphenyl-pyrimidine and 14.1 g (58.1 mmol) potassium phosphate tribasic monohydrate are degassed with argon. 40 ml dioxane,100 ml toluene and 25 ml water are added. The mixture is degassed with argon. 286 mg (0.697 mmol) 2-dicyclohexylphosphino-2',6'-dimethoxybiphenyl (SPhos) and 261 mg (0.116 mmol) palladium (II) acetate are added. The reaction mixture is stirred for 24 h at 100 °C. 40 ml of a 1 % sodium cyanide solution are added and the reaction mixture is refluxed for 1 h. The product (Cpd. A-1)is filtered off and is washed with water and ethanol.
   ¹H NMR (300 MHz, THF-d₈, δ): 8.80 - 8.85 (m, 6H), 8.48-8.55 (m, 8H), 8.00-8.03 (m, 2H), 7.76 (t, J= 5.8 Hz, 2H), 7.51-7.61 (m. 12H).

### Example 2

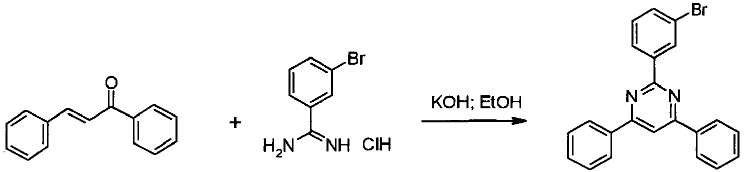

a) 2-(3-bromophenyl)-4,6-diphenyl-pyrimidine is synthesized as described in WO05085387A1. 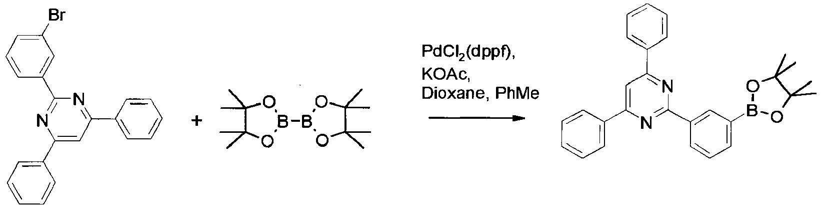
b) 6.00 g (15.5 mmol) 2-(3-bromophenyl)-4,6-diphenyl-pyrimidine, 9.44 g (37.2 mmol) 4,4,5,5-tetramethyl-2-(4,4,5,5-tetramethyl-1,3,2-dioxaborolan-2-yl)-1,3,2-dioxaborolane, 7.60 g (77.5 mmol) potassium acetate are degassed with argon. 60 ml DMF and PdCl₂(dppf) (1,1'-bis(diphenylphosphino)ferrocene-palladium(II) dichloride) are added. The reaction mixture is stirred at 60 °C for 28 h and is then poured onto ice. The water phase is extracted with diethyl ether. The organic phase is dried with magnesium sulfate and the solvent is distilled off. The product is used without purification in ste c). 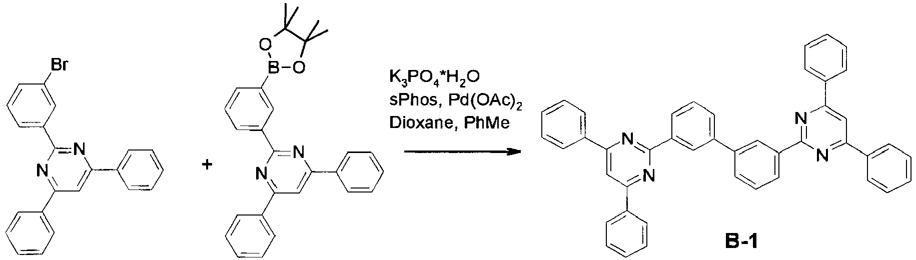
c) 3.50 g (8.06 mmol) of the product of example 2b), 2.31 g g (8.06 mmol) 4-(3-bromophenyl)-2,6-diphenyl-pyrimidine and 9.77 g (40.3 mmol) potassium phosphate tribasic monohydrate are degassed with argon. 30 ml dioxane, 70 ml toluene and 20 ml water are added. 199 mg (0.483 mmol) 2-dicyclohexylphosphino-2',6'-dimethoxybiphenyl (SPhos) and 18 mg (0.081 mmol) palladium (II) acetate are added. The reaction mixture is stirred for 24 h at 100 °C. 40 ml of a 1 % sodium cyanide solution are added and the reaction mixture is refluxed for 1 h. The product is filtered off and is washed with water and ethanol. Column chromatography on silica gel with toluene/ethyl acetate 19/1 results in compound B-1 (yield: 8 %). MS (APCI (pos ): m/z(%): 615 (M⁺¹, 100%).
¹H NMR (300 MHz, THF-d₈,): 9.29 (s, 2H), 8.90 (d, J= 7.9 Hz, 2H), 8.54-8.58 (m, 8H), 8.46 (s, 2H), 8.04 (d, J= 8.0 Hz, 2H), 7.78 (t, J= 8.0 Hz, 2H), 7.60-7.62 (m, 12H).

### Application Example 1

The ITO substrate used as the anode is first cleaned with commercial detergents for LCD production (Deconex^{®} 20NS, and 25ORGAN-ACID^{®} neutralizing agent) and then in an acetone/isopropanol mixture in an ultrasound bath. To eliminate any possible organic residues, the substrate is exposed to a continuous ozone flow in an ozone oven for a further 25 minutes. This treatment also improves the hole injection properties of the ITO. Then Plexcore^{®} OC AJ20-1000 (commercially available from Plextronics Inc.) is spin-coated and dried to form a hole injection layer (~40 nm).

Thereafter, the organic materials specified below are applied by vapor deposition to the clean substrate at a rate of approx. 0.5-5 nm/min at about 10⁻⁷ -10⁻⁹ mbar. As a hole transport and exciton blocker, Ir(dpbic)₃ (for preparation, see Ir complex (7) in the application WO 2005/019373) is applied to the substrate with a thickness of 20 nm, wherein the first 10 nm are doped with MoOₓ (~10%) to improve the conductivity.

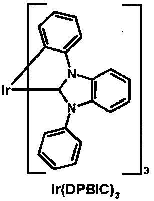

Subsequently, a mixture of 30% by weight of compound 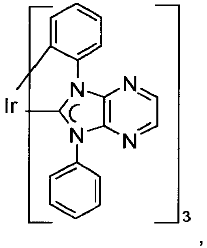 10% by weight of compound 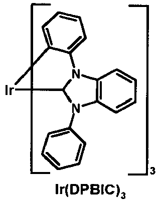 and 60% by weight of compound 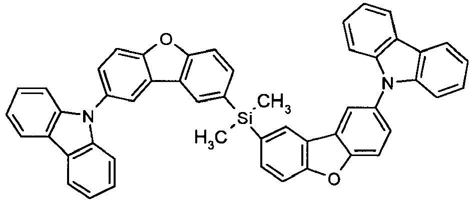 (described in PCT/EP2009/067120) is applied by vapor deposition in a thickness of 20 nm.

Next, compound **A-1** is applied as electron transport layer by vapor deposition in a thickness of 30 nm, as are a 2 nm-thick caesium fluoride layer (electron injection layer) and finally a 100 nm-thick Al electrode.

All prefabricated parts are sealed with a glass lid in an inert nitrogen atmosphere.

### Comparative Application Example 1

Production and construction of an OLED as in the application example 1, except 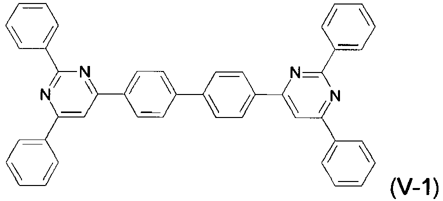 is used instead of compound **A-1.**

To characterize the OLED, electroluminescence spectra are recorded at various currents and voltages. In addition, the current-voltage characteristic is measured in combination with the light output emitted. The light output can be converted to photometric parameters by calibration with a photometer. To determine the lifetime, the OLED is operated at a constant current density and the decrease in the light output is recorded. The lifetime is defined as that time which lapses until the luminance decreases to half of the initial luminance.

| | **ETM** | **Voltage [V]** | **EQE¹⁾ @ 300nits** | **Lifetime²⁾ @ 4000nits** |
|---|---|---|---|---|
| **Appl. Ex. 1** | Cpd. **A-1** | 3.2 | 15.2 % | 131 |
| **Comp. Appl. Ex. 1** | Cpd. **V-1** | 3.4 | 10.1 % | 100 |

1) External quantum efficiency (EQE) is # of generated photons escaped from a substance or a device / # of electrons flowing through it.
2) The measured data of the Comparative Application Example is set to 100 and the data of the Application Examples is specified in relation to that of the Comparative Application Example.

The device of application example 1, where compound **A-1** is used as electron transport material, shows a better external quantum efficiency and a better life time as the device of comparative application example 1, where the compound **V-1** is used as electron transport material.

## Claims

1. A compound of the formula 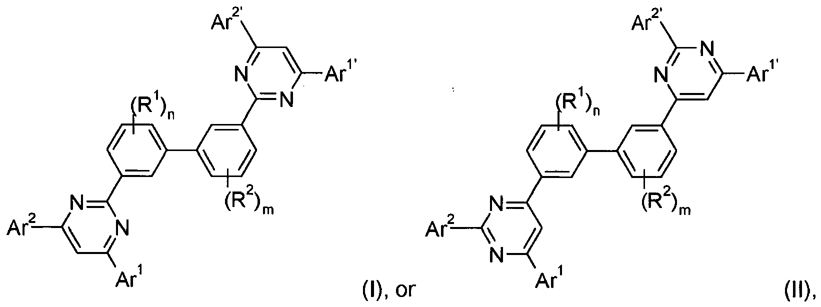 wherein
Ar¹, Ar², Ar^{1'} and Ar^{2'} are independently of each other a C₆-C₂₄aryl group, or a C₂-C₃₀heteroaryl group, which can optionally be substituted by G, a C₄-C₁₈cycloalkyl, a C₆-C₁₀aryl group, a C₆-C₁₀aryl group which is substituted by by C₁-C₈alkyl, a C₂-C₅heteroaryl group, or a C₂-C₅heteroaryl group, which is substituted by by C₁-C₈alkyl, R¹ and R² can be the same or different at each occurrence and are F, CN, NR⁴⁵R⁴⁵', a C₁-C₂₅alkyl group, a C₄-C₁₈cycloalkyl group, a C₁-C₂₅alkoxy group which is substituted by E or interrupted by D, a C₆-C₂₄aryl group, a C₆-C₂₄aryl group which is substituted by G, a C₂-C₃₀heteroaryl group, a C₂-C₃₀heteroaryl group, which is substituted by G,
m and n are 0, 1, 2, 3, or 4,
D is -CO-, -COO-, -S-, -SO-, -SO₂-, -O-, -NR⁶⁵-, -SiR⁷⁰R⁷¹-, -POR⁷²-, -CR⁶³=CR⁶⁴-, or -C≡C-,
E is -OR⁶⁹, -SR⁶⁹, -NR⁶⁵R⁶⁶, -COR⁶⁸, -COOR⁶⁷, -CONR⁶⁵R⁶⁶, -CN, or halogen,
G is E, or C₁-C₂₅alkyl,
R⁴⁵ and R^{45'} are independently of each other a C₁-C₂₅alkyl group, a C₄-C₁₈cycloalkyl group, in which one or more carbon atoms which are not in neighbourhood to each other could be replaced by -NR^{45"}-, -O-, -S-, -C(=O)-O-, or, -O-C(=O)-O-, and/or wherein one or more hydrogen atoms can be replaced by F, a C₆-C₂₄aryl group, or a C₆-C₂₄aryloxy group, wherein one or more carbon atoms can be replaced by O, S, or N, and/or which can be substituted by one or more non-aromatic groups R¹, and
R^{45"} is a C₁-C₂₅alkyl group, or a C₄-C₁₈cycloalkyl group,
R⁶³ and R⁶⁴ are independently of each other H, C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-;
R⁶⁵ and R⁶⁶ are independently of each other C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -0-; or R⁶⁵ and R⁶⁶ together form a five or six membered ring, or ring system;
R⁶⁷ is C₆-C₁₈aryl; C₆-C₁₈aryl which is substituted by C₁-C₁₈a[kyl, or C₁-C₁₈alkoxy; C₁-C₁₈alkyl; or C₁-C₁₈alkyl which is interrupted by -O-,
R⁶⁸ is H; C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by-O-,
R⁶⁹ is C₆-C₁₈aryl; C₆-C₁₈aryl, which is substituted by C₁-C₁₈alkyl, or C₁-C₁₈alkoxy; C₁-C₁₈alkyl; or C₁-C₁₈alkyl which is interrupted by-O-,
R⁷⁰ and R⁷¹ are independently of each other C₁-C₁₈alkyl, C₆-C₁₈aryl, or C₆-C₁₈aryl, which is substituted by C₁-C₁₈alkyl, and
R⁷² is C₁-C₁₈alkyl, C₆-C₁₈aryl, or C₆-C₁₈aryl, which is substituted by C₁-C₁₈alkyl.

2. The compound of formula II according to claim 1, which is a compound of formula 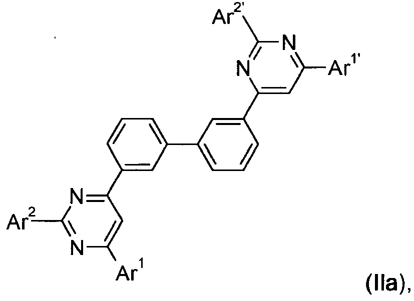 wherein Ar¹, Ar², Ar¹' and Ar^{2'} are as defined in claim 1.

3. The compound according to claim 2, wherein Ar¹, Ar², Ar^{1'} and Ar^{2'} are independently of each other selected from

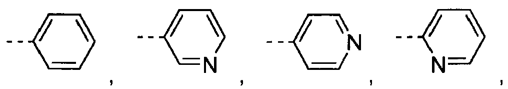

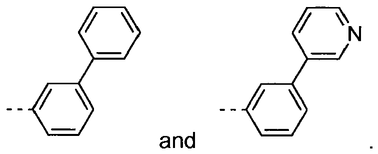

4. The compound according to claim 3: 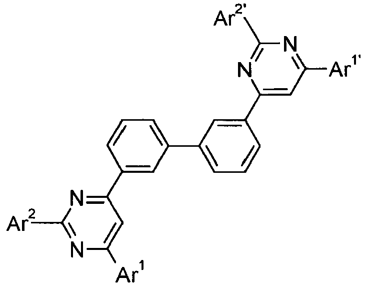
| **Cpd.** | **Ar¹ = Ar^{1'}** | **Ar² = Ar^{2'}** |
|---|---|---|
| **A-1** | 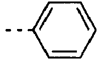 | 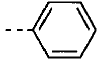 |
| **A-2** | 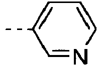 | 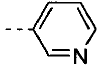 |
| **A3** | 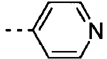 | 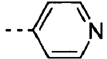 |
| **A-4** | 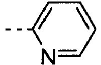 | 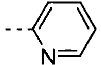 |
| **A-5** | 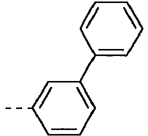 | 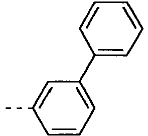 |
| **A-6** | 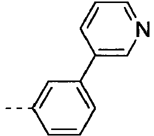 | 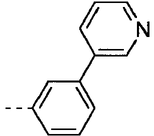 |
| **A-7** | 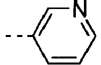 | 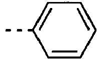 |
| **A-8** | 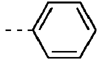 | 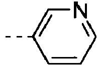 |

5. The compound of formula I according to claim 1, which is a compound of formula 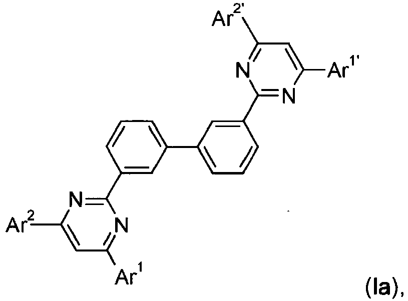 wherein Ar¹, Ar², Ar^{1'} and Ar^{2'} are as defined in claim 1.

6. The compound according to claim 5, wherein Ar¹, Ar², Ar^{1'} and Ar^{2'} are independently of each other selected from

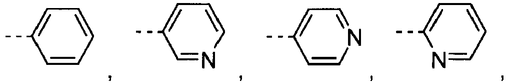

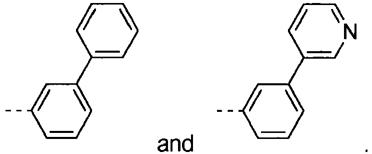

7. The compound according to claim 6: 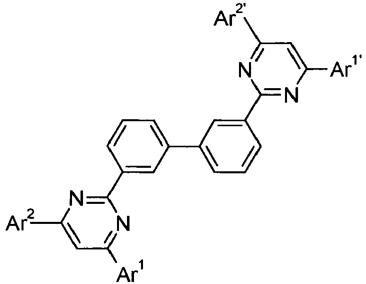
| **Cpd.** | **Ar¹ = Ar^{1'}** | **Ar² = Ar^{2'}** |
|---|---|---|
| **B-1** | 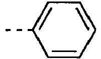 | 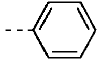 |
| **B-2** | 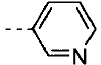 | 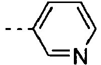 |
| **B-3** | 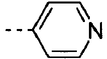 | 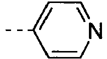 |
| **B-4** | 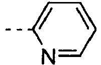 | 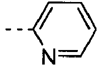 |
| **B-5** | 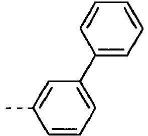 | 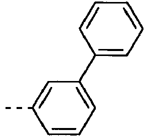 |
| **B-6** | 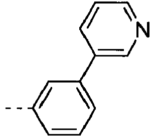 | 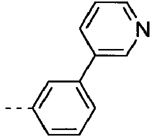 |
| **B-7** | 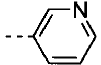 | 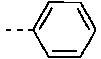 |
| **B-8** | 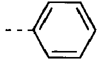 | 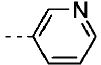 |

8. An electronic device, comprising a compound according to any of claims 1 to 7.

9. The electronic device according to claim 8, which is an electroluminescent device.

10. An electron transport layer, comprising a compound according to any of claims 1 to 7.

11. An apparatus selected from the group consisting of stationary visual display units such as visual display units of computers, televisions, visual display units in printers, kitchen appliances and advertising panels, illuminations, information panels, and mobile visual display units such as visual display units in cellphones, laptops, digital cameras, MP3 players, vehicles and destination displays on buses and trains; illumination units; keyboards; items of clothing; furniture; wallpaper, comprising the organic electronic device according to claim 8, or 9, or the electron transport layer according to claim 10.

12. Use of the compounds of formula I, or II according to claim 1 for electrophotographic photoreceptors, photoelectric converters, organic solar cells (organic photovoltaics), switching elements, organic light emitting field effect transistors (OLEFETs), image sensors, dye lasers and electroluminescent devices.

13. A process for the preparation of compounds of the formula 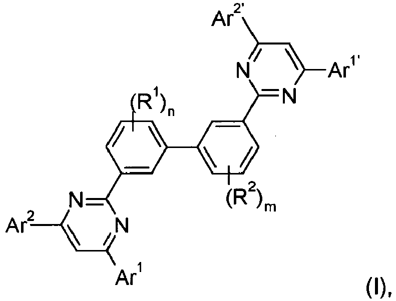 which comprises reacting a compound of formula 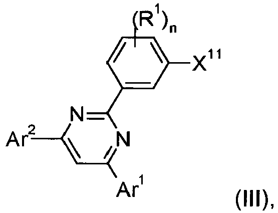 wherein X¹¹ stands for halogen, such as bromo, or iodo, with a compound of formula 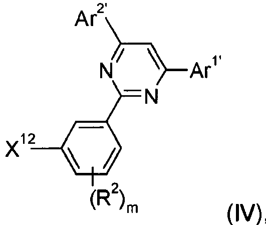 wherein X¹² is -B(OH)₂, -B(OY¹)₂, 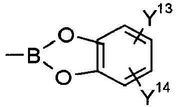 or 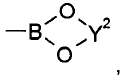 wherein Y¹ is independently in each occurrence a C₁-C₁₈alkylgroup and Y² is independently in each occurrence a C₂-C₁₀alkylene group, such as - CY³Y⁴-CY⁵Y⁶-, or -CY⁷Y⁸-CY⁹Y¹⁰- CY¹¹Y¹²-, wherein Y³, Y⁴, Y⁵, Y⁶, Y⁷, Y⁸, Y⁹, Y¹⁰, Y¹¹ and Y¹² are independently of each other hydrogen, or a C₁-C₁₈alkylgroup, especially -C(CH₃)₂C(CH₃)₂-, -C(CH₃)₂CH₂C(CH₃)₂-, or -CH₂C(CH₃)₂CH₂-, and Y¹³ and Y¹⁴ are independently of each other hydrogen, or a C₁-C₁₈alkylgroup, in the presence of a base and a catalyst in a solvent,
wherein Ar¹, Ar^{1'}, Ar², Ar^{2'}, m, n, R¹ and R² are as defined in claim 1.

14. A process for the preparation of compounds of the formula 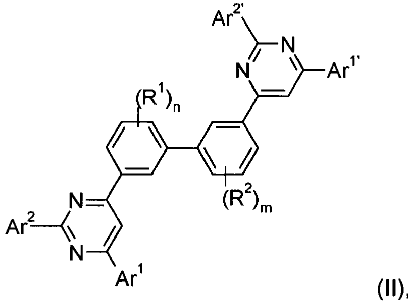 which comprises reacting a compound of formula 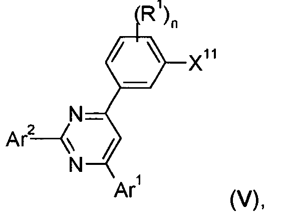 wherein X¹¹ stands for halogen, such as bromo, or iodo, with a compound of formula 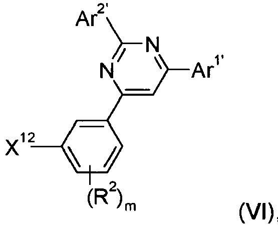 wherein X¹² is -B(OH)₂, -B(OY¹)₂, 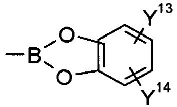 or 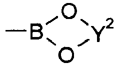 wherein Y¹ is independently in each occurrence a C₁-C₁₈alkylgroup and Y² is independently in each occurrence a C₂-C₁₀alkylene group, such as - CY³Y⁴-CY⁵Y⁶-, or -CY⁷Y⁸-CY⁹Y¹⁰- CY¹¹Y¹²-, wherein Y³, Y⁴, Y⁵, Y⁶, Y⁷, Y⁸, Y⁹, Y¹⁰, Y¹¹ and Y¹² are independently of each other hydrogen, or a C₁-C₁₈alkylgroup, especially -C(CH₃)₂C(CH₃)₂-, -C(CH₃)₂CH₂C(CH₃)₂-, or -CH₂C(CH₃)₂CH₂-, and Y¹³ and Y¹⁴ are independently of each other hydrogen, or a C₁-C₁₈alkylgroup, in the presence of a base and a catalyst in a solvent,
wherein Ar¹, Ar^{1'}, Ar², Ar^{2'}, m, n, R¹ and R² are as defined in claim 1.

## Patentansprüche

1. Verbindung der Formel 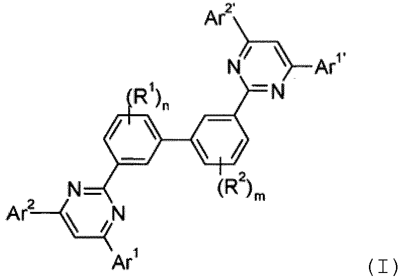 oder 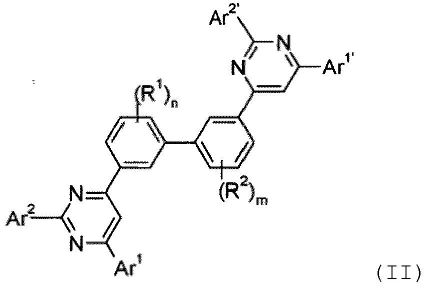 worin
Ar¹, Ar², Ar^{1'} und Ar^{2'} unabhängig voneinander für eine C₆-C₂₄-Arylgruppe oder eine C₂-C₃₀-Heteroarylgruppe, die gegebenenfalls durch G, eine C₄-C₁₈-Cycloalkylgruppe, eine C₆-C₁₀-Arylgruppe, eine C₆-C₁₀-Arylgruppe, die durch C₁-C₈-Alkyl substituiert ist, eine C₂-C₅-Heteroarylgruppe oder eine C₂-C₅-Heteroarylgruppe, die durch C₁-C₈-Alkyl substituiert ist, substituiert sein kann, stehen,
R¹ und R² bei jedem Auftreten gleich oder verschieden sein können und für F, CN, NR⁴⁵R^{45'}, eine C₁-C₂₅-Alkylgruppe, eine C₄-C,₈-Cycloalkyl-gruppe, eine C₁-C₂₅-Alkoxygruppe, die durch E substituiert oder durch D unterbrochen ist, eine C₆-C₂₄-Arylgruppe, eine C₆-C₂₄-Arylgruppe, die durch G substituiert ist, eine C₂-C₃₀-Heteroarylgruppe oder eine C₂-C₃₀-Heteroarylgruppe, die durch G substituiert ist, stehen,
m und n für 0, 1, 2, 3 oder 4 stehen,
D für -CO-, -COO-, -S-, -SO-, -SO₂-, -O-, -NR⁶⁵-, -SiR⁷⁰R⁷¹-, -POR⁷²-, -CR⁶³=CR⁶⁴- oder -C≡C- steht,
E für -OR⁶⁹, -SR⁶⁹, -NR⁶⁵R⁶⁶, -COR⁶⁸ -COOR⁶⁷, -CONR⁶⁵R⁶⁶, -CN oder Halogen steht,
G für E oder C₁-C₂₅-Alkyl steht,
R⁴⁵ und R^{45'} unabhängig voneinander für eine C₁-C₂₅-Alkylgruppe, eine C₄-C₁₈-Cycloalkylgruppe, in der ein oder mehrere Kohlenstoffatome, die einander nicht benachbart sind, durch -NR^{45''}-, -O-, -S-, -C(=O)-O- oder -O-C(=O)-O- ersetzt sein können und/oder worin ein oder mehrere Wasserstoffatome durch F, eine C₆-C₂₄-Arylgruppe oder eine C₆-C₂₄-Aryloxygruppe ersetzt sein können, worin ein oder mehrere Kohlenstoffatome durch 0, S oder N ersetzt sein können und/oder die durch eine oder mehrere nichtaromatische Gruppen R¹ substituiert sein kann, stehen und
R^{45''} für eine C₁-C₂₅-Alkylgruppe oder eine C₄-C₁₈-Cycloalkylgruppe steht,
R⁶³ und R⁶⁴ unabhängig voneinander für H, C₆-C₁₈-Aryl, C₆-C₁₈-Aryl, das durch C₁-C₁₈-Alkyl oder C₁-C₁₈-Alkoxy substituiert ist, C₁-C₁₈-Alkyl oder C₁-C₁₈-Alkyl, das durch -0- unterbrochen ist, stehen; R⁶⁵ und R⁶⁶ unabhängig voneinander für C₆-C₁₈-Aryl, C₆-C₁₈-Aryl, das durch C₁-C₁₈-Alkyl oder C₁-C₁₈-Alkoxy substituiert ist, C₁-C₁₈-Alkyl oder C₁-C₁₈-Alkyl, das durch -0- unterbrochen ist, stehen oder
R⁶⁵ und R⁶⁶ zusammen einen fünf- oder sechsgliedrigen Ring oder ein Ringsystem bilden;
R⁶⁷ für C₆-C₁₈-Aryl, C₆-C₁₈-Aryl, das durch C₁-C₁₈-Alkyl oder C₁-C₁₈-Alkoxy substituiert ist, C₁-C₁₈-Alkyl oder C₁-C₁₈-Alkyl, das durch -0- unterbrochen ist, steht,
R⁶⁸ für H, C₆-C₁₈-Aryl, C₆-C₁₈-Aryl, das durch C₁-C₁₈-Alkyl oder C₁-C₁₈-Alkoxy substituiert ist, C₁-C₁₈-Alkyl oder C₁-C₁₈-Alkyl, das durch -0- unterbrochen ist, steht,
R⁶⁹ für C₆-C₁₈-Aryl, C₆-C₁₈-Aryl, das durch C₁-C₁₈-Alkyl oder C₁-C₁₈-Alkoxy substituiert ist, C₁-C₁₈-Alkyl oder C₁-C₁₈-Alkyl, das durch -0- unterbrochen ist, steht,
R⁷⁰ und R⁷¹ unabhängig voneinander für C₁-C₁₈-Alkyl, C₆-C₁₈-Aryl oder C₆-C₁₈-Aryl, das durch C₁-C₁₈-Alkyl substituiert ist, stehen und
R⁷² für C₁-C₁₈₋Alkyl, C₆-C₁₈-Aryl oder C₆-C₁₈-Aryl, das durch C₁-C₁₈-Alkyl substituiert ist, steht.

2. Verbindung der Formel II nach Anspruch 1, bei der es sich um eine Verbindung der Formel 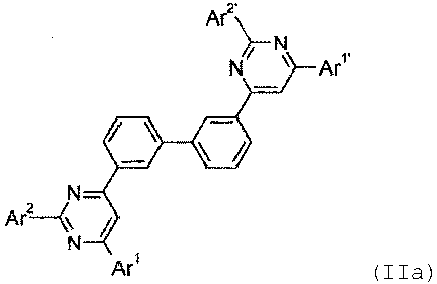 handelt, wobei Ar¹, Ar², Ar^{1'} und Ar²' wie in Anspruch 1 definiert sind.

3. Verbindung nach Anspruch 2, wobei Ar¹, Ar², Ar^{1'} und Ar^{2'} unabhängig voneinander aus 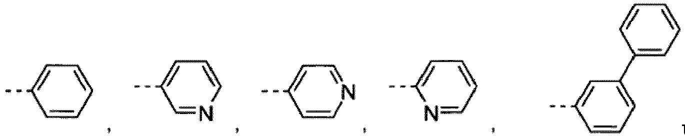 und 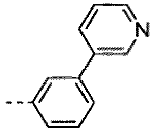 ausgewählt sind.

4. Verbindung nach Anspruch 3: 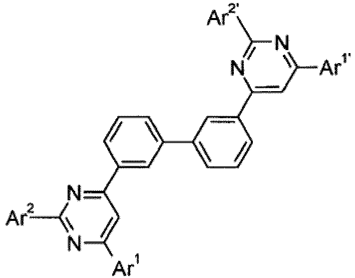
| Verb. | **Ar¹ = Ar^{1'}** | **Ar² = Ar^{2'}** |
|---|---|---|
| **A-1** | 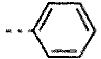 | 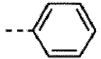 |
| **A-2** | 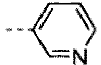 | 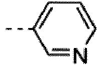 |
| **A-3** | 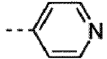 | 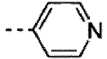 |
| **A-4** | 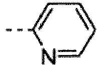 | 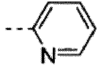 |
| **A-5** | 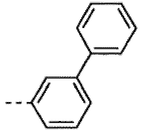 | 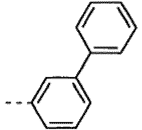 |
| **A-6** | 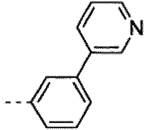 | 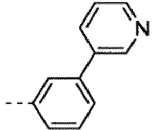 |
| **A-7** | 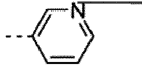 | 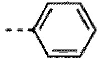 |
| **A-8** | 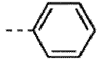 | 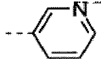 |

5. Verbindung der Formel I nach Anspruch 1, bei der es sich um eine Verbindung der Formel 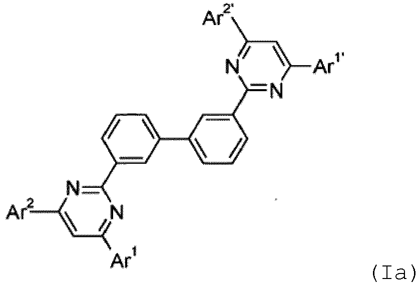 handelt, wobei Ar¹, Ar², Ar^{1'} und Ar^{2'} wie in Anspruch 1 definiert sind.

6. Verbindung nach Anspruch 5, wobei Ar¹, Ar², Ar^{1'} und Ar^{2'} unabhängig voneinander aus 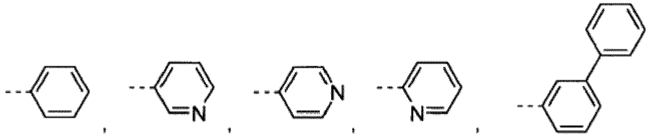 und 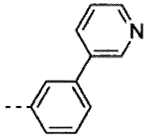 ausgewählt sind.

7. Verbindung nach Anspruch 6: 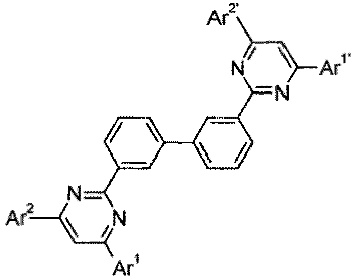
| Verb. | **Ar¹ = Ar^{1'}** | **Ar² = Ar^{2'}** |
|---|---|---|
| **B-1** | 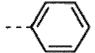 | 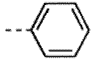 |
| **B-2** | 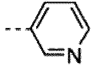 | 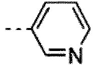 |
| **B-3** | 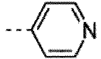 | 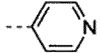 |
| **B-4** | 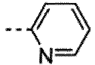 | 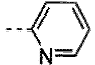 |
| **B-5** | 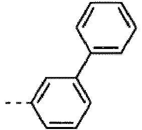 | 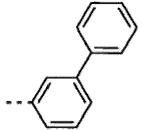 |
| **B-6** | 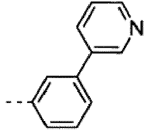 | 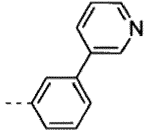 |
| **B-7** | 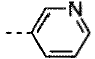 | 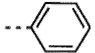 |
| **B-8** | 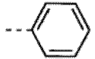 | 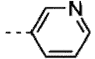 |

8. Elektronische Vorrichtung, umfassend eine Verbindung nach einem der Ansprüche 1 bis 7.

9. Elektronische Vorrichtung nach Anspruch 8, bei der es sich um eine Elektrolumineszenzvorrichtung handelt.

10. Elektronentransportschicht, umfassend eine Verbindung nach einem der Ansprüche 1 bis 7.

11. Apparatur, ausgewählt aus der Gruppe bestehend aus stationären Bildschirmen wie Bildschirmen von Computern, Fernsehern, Bildschirmen in Druckern, Küchengeräten und Reklametafeln, Beleuchtungen, Hinweistafeln und mobilen Bildschirmen wie Bildschirmen in Handys, Laptops, Digitalkameras, MP3-Playern, Fahrzeugen und Zielanzeigen an Bussen und Bahnen; Beleuchtungseinheiten; Tastaturen; Kleidungsstücken; Möbeln; Tapeten, umfassend die organische elektronische Vorrichtung nach Anspruch 8 oder 9 oder die Elektronentransportschicht nach Anspruch 10.

12. Verwendung der Verbindungen der Formel I oder II nach Anspruch 1 für elektrophotographische Photorezeptoren, photoelektrische Umwandler, organische Solarzellen (organische Photovoltaik), Schaltelemente, organische lichtemittierende Feldeffekttransistoren (OLEFETs), Bildsensoren, Farbstofflaser und Elektrolumineszenzvorrichtungen.

13. Verfahren zur Herstellung von Verbindungen der Formel 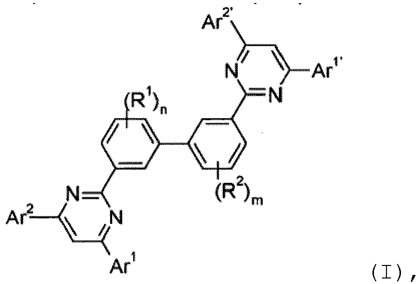 bei dem man eine Verbindung der Formel 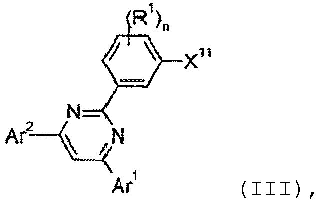 worin X¹¹ für Halogen, wie Brom oder Iod, steht, in Gegenwart einer Base und eines Katalysators in einem Lösungsmittel mit einer Verbindung der Formel 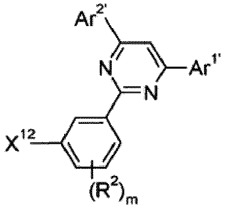 (IV), worin X¹² für -B(OH)₂, -B (OY¹)₂, 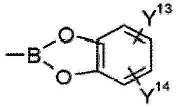 oder 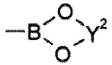 steht, worin Y¹ bei jedem Auftreten unabhängig für eine C₁-C₁₈-Alkylgruppe steht und Y² bei jedem Auftreten unabhängig für eine C₂-C₁₀-Alkylengruppe steht, wie -CY³Y⁴-CY⁵Y⁶- oder -CY⁷Y⁸-CY⁹Y¹⁰-CY¹¹Y¹²-, worin Y³, Y⁴, Y⁵, Y⁶, Y⁷, Y⁸, Y⁹, Y¹⁰, Y¹¹ und Y¹² unabhängig voneinander für Wasserstoff oder eine C₁-C₁₈-Alkylgruppe, insbesondere -C (CH₃) ₂C (CH₃)₂-, -C (CH₃) ₂CH₂C (CH₃)₂- oder -CH₂C (CH₃) ₂CH₂-, stehen und Y¹³ und Y¹⁴ unabhängig voneinander für Wasserstoff oder eine C₁-C₁₈-Alkylgruppe stehen, umsetzt, wobei Ar¹, Ar^{1'}, Ar², Ar^{2'}, m, n, R¹ und R² wie in Anspruch 1 definiert sind.

14. Verfahren zur Herstellung von Verbindungen der Formel 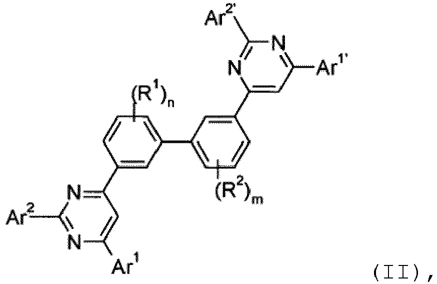 bei dem man eine Verbindung der Formel 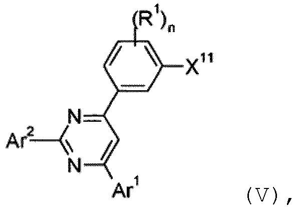 worin X¹¹ für Halogen, wie Brom oder Iod, steht, in Gegenwart einer Base und eines Katalysators in einem Lösungsmittel mit einer Verbindung der Formel 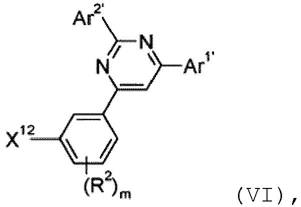 worin X¹² für -B (OH)₂, -B (OY¹)₂, 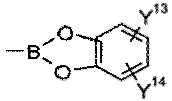 oder 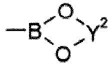 steht, worin Y¹ bei jedem Auftreten unabhängig für eine C₁-C₁₈-Alkylgruppe steht und Y² bei jedem Auftreten unabhängig für eine C₂-C₁₀-Alkylengruppe steht, wie -CY³Y⁴-CY⁵Y⁶- oder -CY⁷Y⁸-CY⁹Y¹⁰-CY¹¹Y¹²-, worin Y³, Y⁴, Y⁵, Y⁶, Y⁷, Y⁸, Y⁹, Y¹⁰, Y¹¹ und Y¹² unabhängig voneinander für Wasserstoff oder eine C₁-C₁₈-Alkylgruppe, insbesondere -C(CH₃)₂C(CH₃)₂-, -C(CH₃₂CH₂C(CH₃)₂- oder -CH₂C(CH₃)₂CH₂-, stehen und Y¹³ und Y¹⁴ unabhängig voneinander für Wasserstoff oder eine C₁-C₁₈-Alkylgruppe stehen, umsetzt, wobei Ar¹, Ar^{1'}, Ar², Ar^{2'}, m, n, R¹ und R² wie in Anspruch 1 definiert sind.

## Revendications

1. Composé de formule 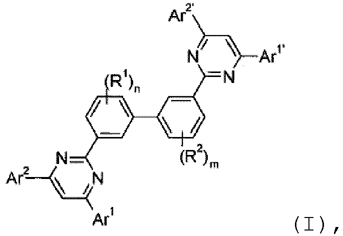 ou 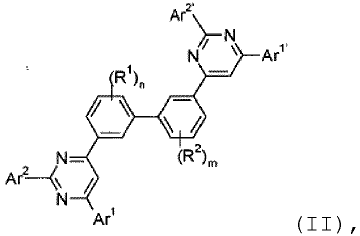 dans lesquelles
Ar¹, Ar², Ar^{1'} et Ar^{2'} représentent indépendamment les uns des autres un groupe aryle en C₆-C₂₄ ou un groupe hétéroaryle en C₂-C₃₀, qui peuvent éventuellement être substitués par G, un cycloalkyle en C₄-C₁₈, un groupe aryle en C₆-C₁₀, un groupe aryle en C₆-C₁₀ qui est substitué par un alkyle en C₁-C₈, un groupe hétéroaryle en C₂-C₅, ou un groupe hétéroaryle en C₂-C₅ qui est substitué par un alkyle en C₁-C₈,
R¹ et R² peuvent être identiques ou différents à chaque occurrence et représentent F, CN, NR⁴⁵R^{45'}, un groupe alkyle en C₁-C₂₅, un groupe cycloalkyle en C₄-C₁₈, un groupe alcoxy en C₁-C₂₅ qui est substitué par E ou interrompu par D, un groupe aryle en C₆-C₂₄, un groupe aryle en C₆-C₂₄ qui est substitué par G, un groupe hétéroaryle en C₂-C₃₀, ou un groupe hétéroaryle en C₂-C₃₀ qui est substitué par G,
m et n valent 0, 1, 2, 3 ou 4,
D représente -CO-, -COO-, -S-, -SO-, -SO₂-, -O-, -NR⁶⁵-, -SiR⁷⁰R⁷¹-, -POR⁷²-, -CR⁶³=CR⁶⁴-, ou -C≡C-,
E représente -OR⁶⁹, -SR⁶⁹, -NR⁶⁵R⁶⁶, -COR⁶⁸, -COOR⁶⁷, -CONR⁶⁵R⁶⁶, -CN, ou un halogène,
G représente E ou un alkyle en C₁-C₂₅,
R⁴⁵ et R^{45'} représentent indépendamment l'un de l'autre un groupe alkyle en C₁-C₂₅, un groupe cycloalkyle en C₄-C₁₈ dans lequel un ou plusieurs atomes de carbone qui ne sont pas voisins les uns des autres pourraient être remplacés par -NR^{45"}-, -O-, -S-, -C(=O)-O- ou -O-C(=O)-O-, et/ou dans lequel un ou plusieurs atomes d'hydrogène peuvent être remplacés par F, un groupe aryle en C₆-C₂₄, ou un groupe aryloxy en C₆-C₂₄ dans lequel un ou plusieurs atomes de carbone peuvent être remplacés par O, S ou N, et/ou qui peut être substitué par un ou plusieurs groupes non aromatiques R¹,
R^{45"} représente un groupe alkyle en C₁-C₂₅ ou un groupe cycloalkyle en C₄-C₁₈,
R⁶³ et R⁶⁴ représentent indépendamment l'un de l'autre H, un aryle en C₆-C₁₈, un aryle en C₆-C₁₈ qui est substitué par un groupe alkyle en C₁-C₁₈ ou alcoxy en C₁-C₁₈, un alkyle en C₁-C₁₈, ou un alkyle en C₁-C₁₈ qui est interrompu par -O-,
R⁶⁵ et R⁶⁶ représentent indépendamment l'un de l'autre un aryle en C₆-C₁₈, un aryle en C₆-C₁₈ qui est substitué par un groupe alkyle en C₁-C₁₈ ou alcoxy en C₁-C₁₈, un alkyle en C₁-C₁₈, ou un alkyle en C₁-C₁₈ qui est interrompu par -O- ; ou bien R⁶⁵ et R⁶⁶ forment ensemble un cycle ou système cyclique à cing ou six chaînons,
R⁶⁷ représente un aryle en C₆-C₁₈, un aryle en C₆-C₁₈ qui est substitué par un groupe alkyle en C₁-C₁₈ ou alcoxy en C₁-C₁₈, un alkyle en C₁-C₁₈, ou un alkyle en C₁-C₁₈ qui est interrompu par -O-,
R⁶⁸ représente H, un aryle en C₆-C₁₈, un aryle en C₆-C₁₈ qui est substitué par un groupe alkyle en C₁-C₁₈ ou alcoxy en C₁-C₁₈, un alkyle en C₁-C₁₈, ou un alkyle en C₁-C₁₈ qui est interrompu par -O-,
R⁶⁹ représente un aryle en C₆-C₁₈, un aryle en C₆-C₁₈ qui est substitué par un groupe alkyle en C₁-C₁₈ ou alcoxy en C₁-C₁₈, un alkyle en C₁-C₁₈, ou un alkyle en C₁-C₁₈ qui est interrompu par -O-,
R⁷⁰ et R⁷¹ représentent indépendamment l'un de l'autre un alkyle en C₁-C₁₈, un aryle en C₆-C₁₈, ou un aryle en C₆-C₁₈ qui est substitué par un alkyle en C₁-C₁₈, et
R⁷² représente un alkyle en C₁-C₁₈, un aryle en C₆-C₁₈, ou un aryle en C₆-C₁₈ qui est substitué par un alkyle en C₁-C₁₈.

2. Composé de formule II selon la revendication 1, qui est un composé de formule 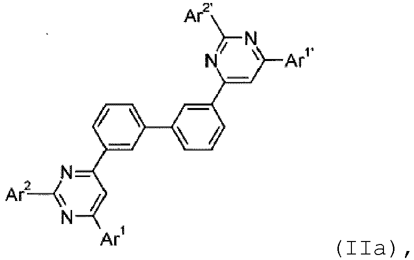 dans laquelle Ar¹, Ar², Ar^{1'} et Ar^{2'} sont tels que définis dans la revendication 1.

3. Composé selon la revendication 2, dans lequel Ar¹, Ar², Ar^{1'} et Ar^{2'} sont indépendamment les uns des autres choisis parmi 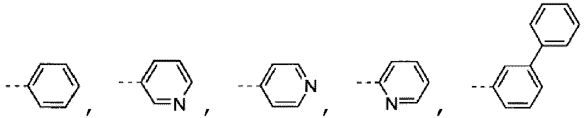 et

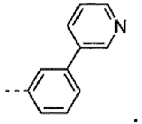

4. Composé selon la revendication 3 : 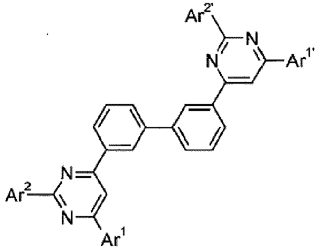
| Composé | Ar¹ = Ar^{1'} | Ar² = Ar^{2'} |
|---|---|---|
| A-1 | 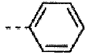 | 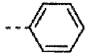 |
| A-2 | 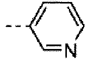 | 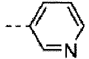 |
| A-3 | 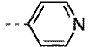 | 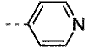 |
| A-4 | 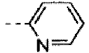 | 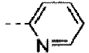 |
| A-5 | 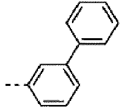 | 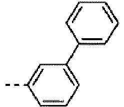 |
| A-6 | 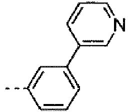 | 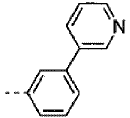 |
| A-7 | 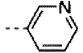 | 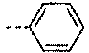 |
| A-8 | 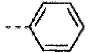 | 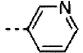 |

5. Composé de formule I selon la revendication 1, qui est un composé de formule 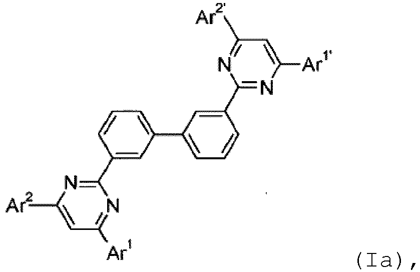 dans laquelle Ar¹, Ar², Ar^{1'} et Ar^{2'} sont tels que définis dans la revendication 1.

6. Composé selon la revendication 5, dans lequel Ar¹, Ar², Ar^{1'} et Ar^{2'} sont indépendamment les uns des autres choisis parmi 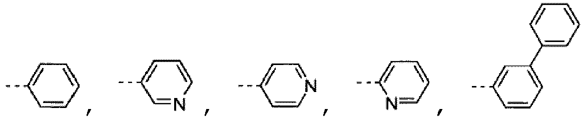 et

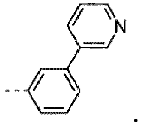

7. Composé selon la revendication 6 : 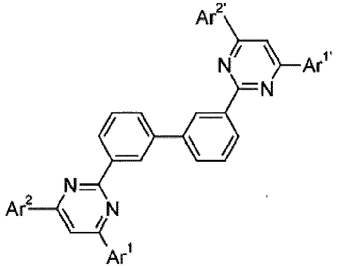
| Composé | Ar¹ = Ar^{1'} | Ar² = Ar^{2'} |
|---|---|---|
| B-1 | 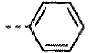 | 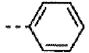 |
| B-2 | 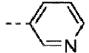 | 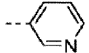 |
| B-3 | 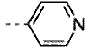 | 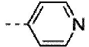 |
| B-4 | 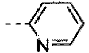 | 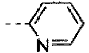 |
| B-5 | 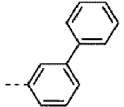 | 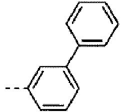 |
| B-6 | 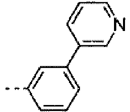 | 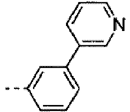 |
| B-7 | 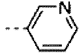 | 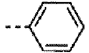 |
| B-8 | 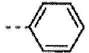 | 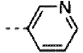 |

8. Dispositif électronique, comprenant un composé selon l'une quelconque des revendications 1 à 7.

9. Dispositif électronique selon la revendication 8, qui est un dispositif électroluminescent.

10. Couche de transport d'électrons, comprenant un composé selon l'une quelconque des revendications 1 à 7.

11. Appareil choisi dans le groupe constitué par les unités d'affichage visuel stationnaires telles que les unités d'affichage visuel des ordinateurs, télévisions, les unités d'affichage visuel dans les imprimantes, les appareils électroménagers et les panneaux publicitaires, les éclairages, les panneaux d'information, et les unités d'affichage visuel mobiles telles que les unités d'affichage visuel dans les téléphones cellulaires, les ordinateurs portables, les caméras numériques, les lecteurs MP3, les véhicules et les affichages de destination sur les bus et les trains ; les unités d'éclairage ; les claviers ; les éléments d'habillage ; le mobilier ; le papier peint, comprenant le dispositif électronique organique selon la revendication 8 ou 9, ou la couche de transport d'électrons selon la revendication 10.

12. Utilisation des composés de formule I ou II selon la revendication 1 pour des photorécepteurs électro-photographiques, des convertisseurs photoélectriques, des cellules solaires organiques (photovoltaïques organiques), des éléments de commutation, des transistors à effet de champ organiques émetteurs de lumière (OLEFET), des capteurs d'images, des lasers à colorants et des dispositifs électroluminescents.

13. Procédé de préparation de composés de formule 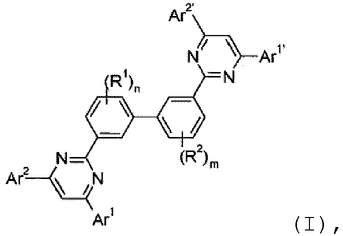 qui comprend la réaction d'un composé de formule 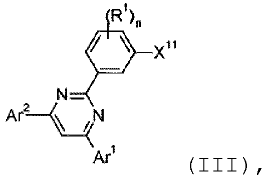 dans laquelle X¹¹ représente un halogène, tel qu'un brome ou un iode, avec un composé de formule 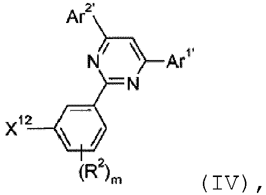 dans laquelle X¹² représente -B(OH)₂, -B(OY¹)₂, 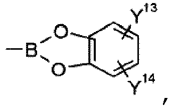 ou 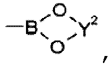 dans lesquels Y¹ représente indépendamment à chaque occurrence un groupe alkyle en C₁-C₁₈ et Y² représente indépendamment à chaque occurrence un groupe alkylène en C₂-C₁₀, tel que -CY³Y⁴-CY⁵Y⁶-, ou -CY⁷Y⁸-CY⁹Y¹⁰-CY¹¹Y¹²-, dans lesquels Y³, Y⁴, Y⁵, Y⁶, Y⁷, Y⁸, Y⁹, Y¹⁰, Y¹¹ et Y¹² représentent indépendamment les uns des autres un hydrogène ou un groupe alkyle en C₁-C₁₈, en particulier -C (CH₃) ₂C (CH₃) ₂-, -C (CH₃) ₂CH₂C (CH₃)₂-, ou -CH₂C(CH₃)₂CH₂-_{,} et Y¹³ et Y¹⁴ représentent indépendamment l'un de l'autre un hydrogène ou un groupe alkyle en C₁-C₁₈, en présence d'une base et d'un catalyseur dans un solvant,
dans lequel Ar¹, Ar^{1'}, Ar², Ar^{2'}, m, n, R¹ et R² sont tels que définis dans la revendication 1.

14. Procédé de préparation de composés de formule 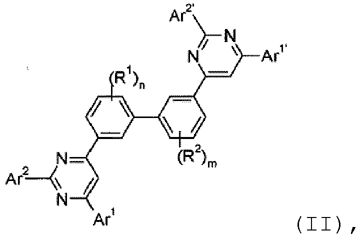 qui comprend la réaction d'un composé de formule 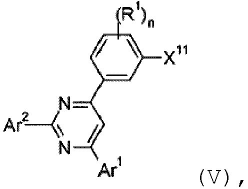 dans laquelle X¹¹ représente un halogène, tel qu'un brome ou un iode, avec un composé de formule 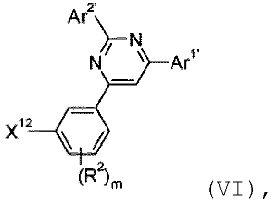 dans laquelle X¹² représente -B(OH)₂, -B(OY¹)₂, 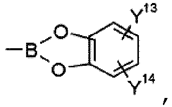 ou 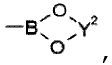 dans lesquels Y¹ représente indépendamment à chaque occurrence un groupe alkyle en C₁-C₁₈ et Y² représente indépendamment à chaque occurrence un groupe alkylène en C₂-C₁₀, tel que -CY³Y⁴-CY⁵Y⁶-, ou -CY⁷Y⁸-CY⁹Y¹⁰-CY¹¹Y¹²-, dans lesquels Y³, Y⁴, Y⁵, Y⁶, Y⁷, Y⁸, Y⁹, Y¹⁰, Y¹¹ et Y¹² représentent indépendamment les uns des autres un hydrogène ou un groupe alkyle en C₁-C₁₈, en particulier -C (CH₃) ₂C (CH₃) ₂-, -C (CH₃) ₂CH₂C (CH₃) ₂-, ou -CH₂C(CH₃)₂CH₂-, et Y¹³ et Y¹⁴ représentent indépendamment l'un de l'autre un hydrogène ou un groupe alkyle en C₁-C₁₈, en présence d'une base et d'un catalyseur dans un solvant,
dans lequel Ar¹, Ar^{1'}, Ar², Ar^{2'}, m, n, R¹ et R² sont tels que définis dans la revendication 1.
